# FASCICULE DE BREVET EUROPEEN

(11) **EP 2 459 734 B1**
(45) Date de publication et mention de la délivrance du brevet: **08.10.2014**
(21) Numéro de dépôt: 10752884.6
(22) Date de dépôt: 28.07.2010
(51) Int. Cl.: C12Q 1/04, C12Q 1/37, G01N 33/84

(54) **NOUVEAUX SUBSTRATS DE PEPTIDASE**
NEUARTIGE PEPTIDASE-SUBSTRATE
NOVEL PEPTIDASE SUBSTRATES

(30) Priorité: 30.07.2009 FR 0903768
(43) Date de publication de la demande: 06.06.2012
(73) Titulaire: Biomérieux, 69280 Marcy L'Etoile (FR)
(72) Inventeur: FABREGA, Olivier, Newcastle Upon Tyne NE3 3GJ (GB); JAMES, Arthur, Cumbria CA13 9 UX (GB); ORENGA, Sylvain, F-01160 Neuville Sur Ain (FR); PERRY, John, Newcastle upon Tyne NE7 7BH (GB); SALWATURA, Vindhya Lakshika, Newcastle Upon Tyne NE4 9JB (GB); STANFORTH, Stephen, Northumberland NE 43 7EH (GB)
(86) Numéro de dépôt international: PCT/FR2010/051597
(87) Numéro de publication internationale: WO 2011/012809

(56) Documents cités:
- WO-A1-98/04735
- WO-A1-99/38995
- WO-A1-2006/030119
- FR-A1- 2 916 763
- MANAFI M ET AL: "FLUOROGENIC AND CHROMOGENIC SUBSTRATES USED IN BACTERIAL DIAGNOSTICS", MICROBIOLOGICAL REVIEWS, AMERICAN SOCIETY FOR MICROBIOLOGY, WASHINGTON, DC, US, vol. 55, no. 3, 1 septembre 1991 (1991-09-01), pages 335-348, XP009020939, ISSN: 0146-0749

## Description

La présente invention concerne de nouveaux composés utilisables à titre d'indicateurs de pH et/ou de substrats enzymatiques pour la détection d'activité peptidase. Ces substrats sont utilisables dans les applications comportant une étape d'hydrolyse enzymatique produisant un signal physico-chimique notamment en microbiologie, biochimie, immunologie, biologie moléculaire, histologie, etc. L'invention concerne également des milieux réactionnels contenant de tels substrats, l'utilisation des substrats ou des milieux pour la détection d'activités peptidases et/ou la différenciation de bactéries à Gram positif par rapport à des bactéries à Gram négatif et des procédés d'utilisation.

Il existe actuellement de très nombreux milieux permettant la détection de microorganismes. Cette détection peut être basée notamment sur l'utilisation de substrats particuliers, spécifiques d'une enzyme du microorganisme que l'on souhaite détecter. D'une manière générale, les substrats synthétiques d'enzymes sont constitués de telle façon que le substrat et le produit de son métabolisme par l'enzyme cible possèdent des propriétés physico-chimiques différentes, permettant de les distinguer et d'évaluer si tout ou partie du substrat a été converti en produit par l'enzyme. Les substrats d'hydrolase, sont généralement constitués d'une première partie spécifique de l'activité enzymatique à révéler, et d'une seconde partie faisant office de marqueur, généralement chromogène ou fluorescent. Ainsi dans le cas des bactéries, par le choix des substrats, selon qu'il y a réaction ou non, il est possible de caractériser la nature d'un microorganisme. Une activité peptidase peut notamment être utilisée pour révéler un groupe, un genre ou une espèce de bactéries. Ainsi, l'activité alanine-aminopeptidase, par exemple, permet de différencier les bactéries à Gram négatif des bactéries à Gram positif.

Des substrats chromogènes enzymatiques pour la détection d'activité peptidase sont connus de l'état de la technique. On peut citer notamment la publication de Manafi (Manafi et al, Microbiol Rev 55(3) : 335-348, 1991), qui est une revue de substrats enzymatiques utilisés en microbiologie. Toutefois, les substrats d'aminopeptidase décrits libèrent par hydrolyse des composés diffusant dans le milieu (beta-naphtylamine, 7-amino-4-methylcoumarine). De ce fait, en milieu réactionnel hétérogène (colonies sur boites de Petri, coupe histologique...), il n'est pas possible de localiser précisément le lieu de l'hydrolyse. On peut citer également les substrats décrits dans les demandes de brevet WO 98/04735 et WO 99/38995 déposées par la Demanderesse. Toutefois, bien que ces substrats diffusent peu en milieu de culture, ils présentent certains inconvénients : leur synthèse est difficile, la pureté est réduite, les rendements faibles et ils sont toxiques vis-à-vis de certains micro-organismes. D'autres substrats enzymatiques pour la détecion d'activité peptidase sont décrits dans la demande de brevet FR 2916763.

La présente invention propose donc l'utilisation de nouveaux composés, soit à titre d'indicateurs de pH, soit à titre de substrats de peptidase, permettant la détection de microorganismes. Comparativement aux substrats existants, ces nouveaux composés sont de synthèse aisée, et peuvent être utilisés notamment en milieux gélifiés pour la détection de micro-organismes car ils produisent une coloration ne diffusant peu ou pas dans le milieu réactionnel. Dans le cadre d'une utilisation comme substrat enzymatique, cela permet de repérer une colonie ou une organelle exprimant une activité peptidase parmi d'autres ne l'exprimant pas.

Avant d'aller plus avant dans la description de l'invention, les définitions ci dessous sont données afin de faciliter l'exposé de l'invention.

Par substrat enzymatique, on entend un substrat pouvant être hydrolysé par une enzyme en un produit permettant la détection, directe ou indirecte d'un microorganisme, d'une cellule ou d'une organelle. Ce substrat comprend notamment une première partie spécifique de l'activité enzymatique à révéler et une seconde partie faisant office de marqueur.

Les composés selon l'invention utilisés comme substrats sont adaptés à une utilisation en cytométrie en flux, car le produit de l'hydrolyse restant principalement localisé dans la cellule exprimant l'activité enzymatique, il est possible de compter spécifiquement les cellules exprimant cette activité, voire de les séparer du reste de l'échantillon.

Les composés selon l'invention utilisés comme substrats sont également bien adaptés à une utilisation en histoenzymologie, car le produit d'hydrolyse restant principalement localisé sur le lieu de l'hydrolyse, il est possible d'identifier spécifiquement les cellules ou organelles exprimant cette activité au sein d'un tissu.

Du fait de leur faible toxicité, les composés selon l'invention sont bien adaptés, respectivement comme indicateurs de pH, ou pour le suivi d'activité peptidase en culture cellulaire.

Les composés selon l'invention sont particulièrement bien adaptés à une utilisation en milieu de détection et/ou d'identification car ils produisent une coloration ou une fluorescence ne diffusant pas dans le milieu réactionnel.

Dans la présente demande, le terme coloration est employé pour couvrir une coloration, absorption de lumière dans le spectre visible, ou une fluorescence, absorption à une longueur d'onde (λₑₓ) et émission à une longueur d'onde supérieure (λₑₘ, λₑₘ > λₑₓ). Les composés de l'invention peuvent être salifiés, c'est à dire sous forme de sel tel que chlorure, bromure, iodure ou trifluoroacétate.

Par indicateur de pH, on entend une substance chimique dont la couleur et/ou la fluorescence varie en fonction des modifications de pH du milieu, lesdites modifications étant liées ou non au métabolisme du ou des microorganismes en croissance sur ledit milieu.

Par peptidase, on entend une enzyme capable de cliver par hydrolyse le groupement amide formé entre le reste acyle d'un peptide et une amine primaire. Par aminopeptidase, on entend une enzyme capable de cliver par hydrolyse le groupement amide formé entre un acyle d'acide aminé et une amine primaire. Dans la présente demande, le terme « peptidase » peut désigner, selon les cas, tant une peptidase qu'une aminopeptidase telles que définies précédemment.

Par peptide, on entend une chaîne peptidique comprenant de 1 à 10 acides aminés, préférentiellement de 1 à 4 acides aminés. Préférentiellement, le peptide est un di-Alanine ou tri-Alanine. Par acide aminé, on entend tout acide aminé connu de l'homme du métier, naturel ou non. Selon un mode particulier de réalisation de l'invention, l'acide aminé est une beta-Alanine ou L-Alanine ou D-Alanine, ou une Glycine, Pyrroglutamyl, ...

Ledit peptide peut comprendre un agent de blocage en son extrémité N terminale. Les agents de blocage selon l'invention comprennent tout agent de blocage connu de l'homme du métier qui est capable de protéger les amines. A titre d'exemple, on peut citer le t-Butoxycarbonyle (N-tBOC), le 9-Fluorenyloxycarbonyle, un agent de solubilisation tel que le Succinyle, ou bien un acide aminé non métabolisable, c'est-à-dire non naturel, tel que l'acide pipécolique ou la forme D d'un acide aminé, tel que la D-Phénylalanine. Les agents de blocage ne sont pas systématiquement présents dans les composés de l'invention.

Par groupement alkyle, on entend une chaîne de groupements hydrocarbonés saturés, tel que notamment un alkyle en C₁-C₆, c'est a dire un alkyle droit ou ramifié ayant de 1 à 6 atomes de carbone. A titre d'exemple, on peut citer le méthyle, l'éthyle, le propyle, l'iso-propyle, le butyle, le t-butyle, le pentyle, l'iso-pentyle et l'hexyle.

Par groupement aryle, on entend un groupement fonctionnel (ou substituant) qui dérive d'un noyau aromatique tel que notamment un noyau aromatique en C₆-C₁₀, notamment phényle, benzyle, 1-naphtyle ou 2-naphtyle.

Par groupement carboxyle, on entend notamment un groupe fonctionnel composé d'un atome de carbone, lié par une double liaison à un premier atome d'oxygène, et par une liaison simple à un second atome d'oxygène, lui-même chargé négativement ou relié à un atome d'hydrogène. En fonction du pKₐ de la molécule et du pH du milieu, le groupement carboxyle peut être sous forme ionisée, c'est à dire sans H lié au second atome d'oxygène, qui est alors chargé négativement.

Par milieu réactionnel, on entend un milieu comprenant tous les éléments nécessaires à l'expression d'un métabolisme et/ou à la croissance de microorganismes, d'une cellule ou d'une organelle. Ce milieu réactionnel peut être utilisé en cytométrie en flux, histoenzymologie, culture cellulaire ... ou en tant que milieu de détection et/ou d'identification de microorganismes.

Le milieu réactionnel peut comprendre un ou plusieurs éléments en combinaison, tels que des acides aminés, des peptones, des hydrates de carbone, des nucléotides, des minéraux, des vitamines, des antibiotiques, des tensioactifs, des tampons, des sels de phosphate, d'ammonium, de sodium, de métaux.

Le milieu peut comprendre également un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant ...

Le milieu réactionnel peut être solide, semi-solide ou liquide. Par milieu solide, on entend par exemple un milieu gélifié. L'agar est l'agent gélifiant traditionnel en microbiologie pour la culture des microorganismes, mais il est possible d'utiliser de la gélatine ou de l'agarose. Un certain nombre de préparations sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).

Le milieu réactionnel peut être un milieu de détection et/ou d'identification, c'est à dire un milieu de révélation ou un milieu de culture et de révélation. Dans le premier cas, la culture des microorganismes est effectuée avant ensemencement et, dans le deuxième cas, le milieu de détection et/ou d'identification constitue également le milieu de culture.

Par échantillon biologigue, on entend un échantillon clinique, issu d'un prélèvement de liquide biologique ou un échantillon alimentaire, issu de tout type d'aliment ou un échantillon cosmétique ou pharmaceutique issu de toute préparation cosmétique ou pharmaceutique. Cet échantillon peut être ainsi liquide ou solide et on peut citer d'une manière non limitative, un échantillon clinique de sang, de plasma, d'urines, de fécès, de prélèvements de nez, de gorges, de peaux, de plaies, de liquide céphalo-rachidien, un échantillon alimentaire d'eau, de boissons tels que le lait, un jus de fruits; de yaourt, de viande, d'oeufs, de légumes, de mayonnaise, de fromage ; de poisson..., un échantillon alimentaire issu d'une alimentation destinée aux animaux, tel que notamment un échantillon issu de farines animales. L'échantillon peut également être issu d'un prélèvement de l'environnement clinique, d'élevage ou de production alimentaire, cosmétique ou pharmaceutique. Par prélèvement d'environnement, on entend notamment un prélèvement de surface, de liquide, de matière première ou de produit. Par échantillon, on entend donc le prélèvement en lui même (écouvillon, selles, aliments, ...) aussi bien que des colonies de microorganismes issus dudit prélèvement (par exemple après isolement sur un milieu de culture gélifié, ou dans un bouillon d'enrichissement ensemencé avec ledit prélèvement).

Au sens de la présente invention, le terme microorganisme recouvre les bactéries, les levures, les moisissures et plus généralement, les organismes généralement unicellulaires, invisibles à l'oeil nu, qui peuvent être multipliés et manipulés en laboratoire.

A titre de bactéries à Gram négatif, on peut citer les bactéries des genres suivants : *Pseudomonas, Escherichia, Salmonella, Shigella, Enterobacter, Klebsiella, Serratia, Proteus, Campylobacter, Haemophilus, Morganella, Vibrio, Yersinia, Acinetobacter, Branhamella, Neisseria, Burkholderia, Citrobacter, Hafnia, Edwardsiella, Aeromonas, Moraxella, Pasteurella, Providencia, Actinobacillus, Alcaligenes, Bordetella, Cedecea, Erwinia, Pantoea, Ralstonia, Stenotrophomonas, Xanthomonas* et *Legionella.*

A titre de bactéries à Gram positif, on peut citer les bactéries des genres suivants : *Aerococcus, Enterococcus, Streptococcus, Staphylococcus, Bacillus, Lactobacillus. Listeria, Clostridium, Gardnerella, Kocuria, Lactococcus, Leuconostoc, Micrococcus, Falkamia, Gemella, Pediococcus, Mycobacterium* et *Corynebacterium.*

A titre de levures, on peut citer les levures des genres suivants : *Candida, Cryptococcus, Saccharomyces* et *Trichosporon.*

Préférentiellement, le microorganismes est choisi parmi *Escherichia coli, Serratia marcescens, Enterobacter cloacae, Salmonella typhimurium, Providencia rettgeri. Yersinia enterocolitica, Pseudomonas aeruginosa, Bacillus subtilis, Listeria monocytogenes, Staphylococcus aureus, Streptococcus pyogenes, Enterococcus faecalis.*

A ce titre l'invention concerne l'utilisation d'un composé de la formule (I) suivante, comme substrat enzymatique pour la détection d'une activité peptidase et/ou une variation de pH : 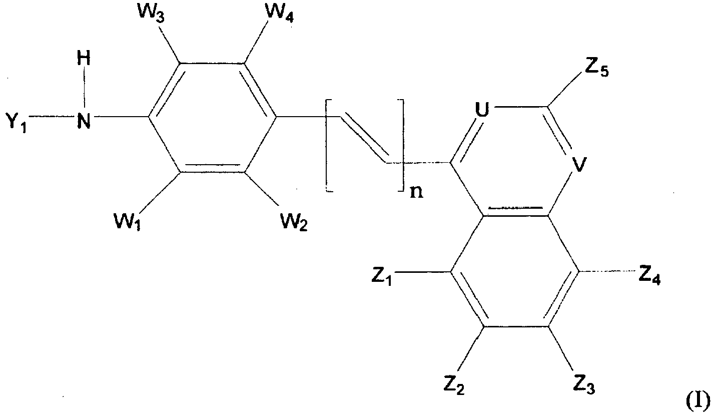 selon laquelle :
- Y₁ est un peptide, H ou un alkyl
- W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci
- n = 0, 1 ou 2
- U est N ou N⁺R et V est CZ₆ N ou N⁺R ou bien V est N ou N⁺R et U est CZ₆,
- R est H, alkyle, aralkyle, aryle, alcanoïque ou alkylsulfonique
- Z₁, Z₂, Z₃, Z₄, Z₅ et Z₆, sont indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels.

Lorsque ledit composé de formule (I) est utilisé pour détecter uniquement une variation de pH, Y₁ est H ou un alkyl.

Lorsque ledit composé de formule (I) est utilisé pour la détection d'une activité peptidase, Y₁ est un peptide. Préférentiellement U ou V est N.

Lorsque ledit composé de formule (I) est utilisé pour la détection d'une activité peptidase et d'une variation de pH, Y₁ est un peptide. Préférentiellement U ou V est N. Selon un mode préféré de réalisation de l'invention, Y₁ est un peptide, préférentiellement choisi parmi l'alanine, l'asparagine, la glutamine, la tyrosine et Tri-alanyl.

Selon un mode préféré de réalisation de l'invention, n = 1

Selon un mode préféré de réalisation de l'invention, W₁, W₂, W₃ et W₄ sont indépendamment H

Selon un mode préféré de réalisation de l'invention, U est CH et V est N ou N⁺R, préférentiellement N⁺CH3

Selon un mode préféré de réalisation de l'invention, V est CH et U est N ou N⁺R, préférentiellement N⁺CH3

Selon un mode préféré de réalisation de l'invention, Z₁, Z₂, Z₃, Z₄, Z₅, et Z₆ sont indépendamment H

Selon un mode préféré de réalisation de l'invention, L-Alanyl-4-(4'-amidophenyl)-2-méthyl-quinoline, L-Alanyl-4-(4'-amidostyryl)-N-benzyl-quinolinium TFA, L-Glutamyl-4-(4'-amidostyryl)-N-benzyl-quinolinium TFA, beta-Alanyl-4-(4'-amidostyryl)-N-benzyl-quinolinium TFA, L-Alanyl-4-(4'-amidostyryl)-N-methylquinolinium TFA, D-Alanine-N-méthyl-4-(4'-aminostyryl)-quinolinium (dichlorure), L-Alanyl-2-(4'-amidostyryl)-N-méthyl-quinolinium, Z-Alanyl-2-(4'-amidostyryl)-N-méthyl-quinolinium, L-Alanyl-4-(4'-amidostyryl)-N-méthyl-quinolinium (dichlorure), L-Alanyl-L-alanyl-4-(4'-amidostyryl)-N-méthyl-quinolinium (dichlorure), L-Alanyl-L-alanyl-L-alanyl-4-(4'-amidostyryl)-N-méthyl-quinolinium (dichlorure), γ-Aminobutyryl-4-(4'-amidostyryl)-N-methylquinolinium di-Cl, L-Asparaginyl-4-(4'-amidostyryl)-N-méthyl-quinolinium-bis-TFA, α-Aspartyl-4-(4'-amidostyryl)-N-methylquinolinium dichloride, β-Aspartyl-4-(4'-amidostyryl)-N-methylquinolinium dichloride, Glycyl-4-(4'-amidostyryl)-N-méthyl-quinolinium (dichlorure), L-gamma-Glutamyl-4-(4'-amidostyryl)-N-méthyl-quinolinium (dichlorure), Glycyl-4-(4'-amidostyryl)-N-méthyl-quinolinium chloride, L-Leucyl-4-(4'-amidostyryl)-N-méthyl-quinolinium dihydrochloride, L-Methionyl-4-(4'-amidostyryl)-N-methyl-quinolinium di-Cl, Pyroglutamyl-4-(4'-amidostyryl)-N-méthyl-quinolinium iodide, Sarcosinyl-4-(4'-amidostyryl)-N-methyl-quinolinium dichloride, L-Tryptophanyl-4-(4-amidostyryl)-N-méthyl-quinolinium dihydrochloride, L-Tyrosyl-4-(4'-amidostyryl)-N-méthyl-quinolinium dihydrochloride, beta-Alanyl-2-(4'-amidostyryl)-N-methyl-quinolinium TFA, L-Prolyl-2-(4'-amidostyryl)-N-méthyl-quinolinium (HBr), Z-Arginyl-2-(4'-amidostyryl)-N-méthyl-quinolinium, L-Pyroglutamyl -2-(4'-amidostyryl)-N-méthyl-quinolinium, L-Alanyl-4-(4'-amidostyryl)quinoline TFA, β-Alanyl-4-(4'-amidostyryl)-quinoline, L-Glutamyl-4-(4'-amidostyryl)quinoline TFA, Glycyl-4-(4'-amidostyryl)quinoline TFA, Pyroglutamyl-4-(4'-amidostyryl) quinoline, L-Alanyl-2-(4'-amidostyryl)-quinoline, β-Alanyl-2-(4'-amidostyryl)-quinoline, Glycyl-2-(4'-amidostyryl)quinoline TFA, L-Alanyl-2-(2'-pyridyl)-4-(4"-amidostyryl)-quinoline Selon un mode préféré de réalisation de l'invention, ledit composé est choisi parmi : β-Alanyl-2-(4'-amidostyryl)-quinoline (β-Ala-ASQ), L-Tyrosyl-4-(4'-amidostyryl)-N-méthyl-quinolinium dihydrochloride (Tyr-ASQM⁺), L-Asparaginyl-4-(4'-amidostyryl)-N-méthyl-quinolinium-bis-TFA (Asn-ASQM⁺), L-gamma-Glutamyl-4-(4'-amidostyryl)-N-méthyl-quinolinium (dichlorure) (γ-Glu-ASQM⁺), L-Alanyl-L-alanyl-L-alanyl-4-(4'-amidostyryl)-N-méthyl-quinolinium (dichlorure) (Tri-Ala-ASQM⁺)

L'invention concerne également un procédé pour la détection chez des micro-organismes d'une activité peptidase et/ou d'une variation de pH, caractérisé en ce qu'il comprend, ou est constitué par les étapes suivantes :
a) disposer d'un milieu de détection et/ou d'identification comprenant composé de la formule (I) suivante : 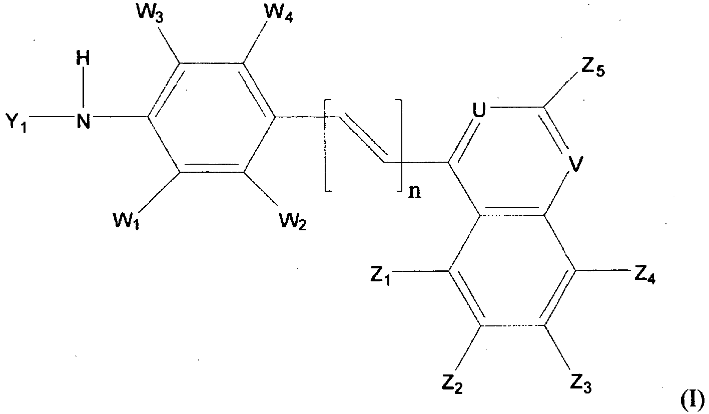 selon laquelle :
   - Y₁ est un peptide, H ou un alkyl
   - W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci
   - n = 0, 1 ou 2
   - U est N ou N⁺R et V est CZ₆ N ou N⁺R ou bien V est N ou N⁺R et U est CZ₆,
   - R est H, alkyle, aralkyle, aryle, alcanoïque ou alkylsulfonique
   - Z₁, Z₂, Z₃, Z₄ et Z₅, sont indépendamment H, Br, Cl, F, I, alkyle, aryle, alkoxy, perfluoroalkyle, nitro, cyano, carboxyle, sulfonyle, incluant les esters ou amides de carboxyle ou sulfonyle
   et leurs sels.
b) ensemencer le milieu avec un échantillon biologique à tester,
c) laisser incuber, et
d) révéler la présence d'au moins une activité peptidase ou une variation de pH

Lorsque ledit procédé est un procédé pour détecter uniquement une variation de pH, Y₁ est H ou un alkyl.

Lorsque ledit procédé est un procédé pour la détection chez des micro-organismes d'une activité peptidase, Y₁ est un peptide. Préférentiellement U ou V est N.

Lorsque ledit procédé est un procédé pour la détection chez des micro-organismes d'une activité peptidase et d'une variation de pH, Y₁ est un peptide. Préférentiellement U ou V est N.

Selon un mode préféré de réalisation de l'invention, Y₁ est un peptide, préférentiellement choisi parmi l'alanine, l'asparagine, la glutamine, la tyrosine Selon un mode préféré de réalisation de l'invention, n = 1

Selon un mode préféré de réalisation de l'invention, W₁, W₂, W₃ et W₄ sont indépendamment H

Selon un mode préféré de réalisation de l'invention, U est CH et V est N ou N⁺R, préférentiellement N⁺CH3

Selon un mode préféré de réalisation de l'invention, V est CH et U est N ou N⁺R, préférentiellement N⁺CH3

Selon un mode préféré de réalisation de l'invention, Z₁, Z₂, Z₃ et Z₄, Z₅, et Z₆ sont indépendamment H

Selon un mode préféré de réalisation de l'invention, L-Alanyl-4-(4'-amidophenyl)-2-méthyl-quinoline, L-Alanyl-4-(4'-amidostyryl)-N-benzyl-quinolinium TFA, L-Glutamyl-4-(4'-amidostyryl)-N-benzyl-quinolinium TFA, beta-Alanyl-4-(4'-amidostyryl)-N-benzyl-quinolinium TFA, L-Alanyl-4-(4'-amidostyryl)-N-methyl-quinolinium TFA, D-Alanine-N-méthyl-4-(4'-aminostyryl)-quinolinium (dichlorure), L-Alanyl-2-(4'-amidostyryl)-N-méthyl-quinolinium, Z-Alanyl-2-(4'-amidostyryl)-N-méthyl-quinolinium, L-Alanyl-4-(4'-amidostyryl)-N-méthyl-quinolinium (dichlorure), L-Alanyl-L-alanyl-4-(4'-amidostyryl)-N-méthyl-quinolinium (dichlorure), L-Alanyl-L-alanyl-L-alanyl-4-(4'-amidostyryl)-N-méthyl-quinolinium (dichlorure), γ-Aminobutyryl-4-(4'-amidostyryl)-N-methylquinolinium di-Cl, L-Asparaginyl-4-(4'-amidostyryl)-N-méthyl-quinolinium-bis-TFA, α=Aspartyl-4-(4'-amidostyryl)-N-methylquinolinium dichloride, β-Aspartyl-4-(4'-amidostyryl)-N-methylquinolinium dichloride, Glycyl-4-(4'-amidostyryl)-N-méthyl-quinolinium (dichlorure), L-gamma-Glutamyl-4-(4'-amidostyryl)-N-méthyl-quinolinium (dichlorure), Glycyl-4-(4'-amidostyryl)-N-méthyl-quinolinium chloride, L-Leucyl-4-(4'-amidostyryl)-N-méthyl-quinolinium dihydrochloride, L-Methionyl-4-(4'-amidostyryl)-N-methyl-quinolinium di-CI, Pyroglutamyl-4-(4'-amidostyryl)-N-méthyl-quinolinium iodide, Sarcosinyl-4-(4'-amidostyryl)-N-methyl-quinolinium dichloride, L-Tryptophanyl-4-(4-amidostyryl)-N-méthyl-quinolinium dihydrochloride, L-Tyrosyl-4-(4'-amidostyryl)-N-méthyl-quinolinium dihydrochloride, beta-Alanyl-2-(4'-amidostyryl)-N-methyl-quinolinium TFA, L-Prolyl-2-(4'-amidostyryl)-N-méthyl-quinolinium (HBr), Z-Arginyl-2-(4'-amidostyryl)-N-méthyl-quinolinium, L-Pyroglutamyl -2-(4'-amidostyryl)-N-méthyl-quinolinium, L-Alanyl-4-(4'-amidostyryl)quinoline TFA, β-Alanyl-4-(4'-amidostyryl)-quinoline, L-Glutamyl-4-(4'-amidostyryl)quinoline TFA, Glycyl-4-(4'-amidostyryl)quinoline TFA, Pyroglutamyl-4-(4'-amidostyryl) quinoline, L-Alanyl-2-(4'-amidostyryl)-quinoline, β-Alanyl-2-(4'-amidostyryl)-quinoline, Glycyl-2-(4'-amidostyryl)quinoline TFA, L-Alanyl-2-(2'-pyridyl)-4-(4"-amidostyryl)-quinoline Selon un mode préféré de réalisation de l'invention, ledit composé est choisi parmi : β-Alanyl-2-(4'-amidostyryl)-quinoline (β-Ala-ASQ), L-Tyrosyl-4-(4'-amidostyryl)-N-méthyl-quinolinium dihydrochloride (Tyr-ASQM⁺), L-Asparaginyl-4-(4'-amidostyryl)-N-méthyl-quinolinium-bis-TFA (Asn-ASQM⁺), L-gamma-Glutamyl-4-(4'-amidostyryl)-N-méthyl-quinolinium (dichlorure) (γ-Glu-ASQM⁺), L-Alanyl-L-alanyl-L-alanyl-4-(4'-amidostyryl)-N-méthyl-quinolinium (dichlorure) (Tri-Ala-ASQM⁺)

L'ensemencement des microorganismes peut être réalisé par toutes les techniques d'ensemencement connues de l'homme du métier. Une étape d'incubation peut être réalisée à une température pour laquelle l'activité enzymatique que l'on souhaite détecter est optimale, que l'homme du métier peut choisir aisément selon l'activité enzymatique à détecter. L'étape d) peut s'effectuer par un examen visuel, par colorimétrie ou fluorimétrie. Lors de l'étape d), on peut révéler la présence de l'activité peptidase ou la variation de pH, seule ou en combinaison avec au moins une autre activité enzymatique.

Préférentiellement, le microorganismes est choisi parmi *Escherichia coli, Serratia marcescens, Enterobacter cloacae, Salmonella typhimurium, Providencia rettgeri, Yersinia enterocolitica, Pseudomonas aeruginosa, Bacillus subtilis, Listeria monocytogenes, Staphylococcus aureus, Streptococcus pyogenes, Enterococcus faecalis.*

La description concerne également un procédé pour la différenciation chez des bactéries de leur appartenance aux bactéries à Gram positif ou aux bactéries à Gram négatif, caractérisé en ce qu'il comprend par les étapes suivantes :
a) disposer d'un milieu de détection et/ou d'identification comprenant composé de la formule (I) suivante, comme substrat enzymatique pour la détection d'une activité peptidase et/ou d'une variation de pH : 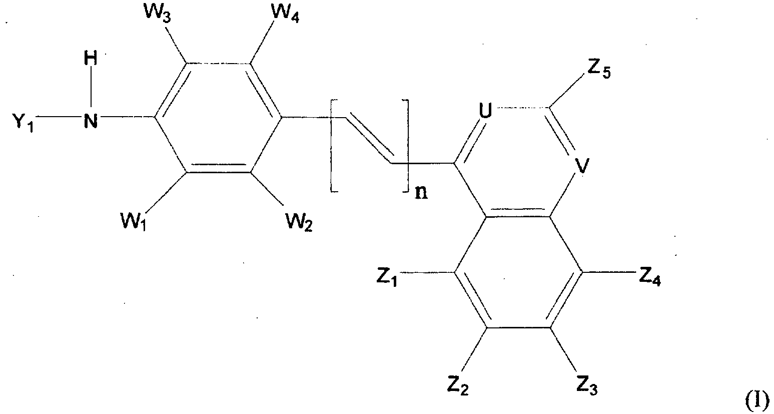 selon laquelle :
   - Y, est un peptide, H ou un alkyl
   - W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci
   - n = 0, 1 ou 2
   - U est N ou N⁺R et V est CZ₆ N ou N⁺R ou bien V est N ou N⁺R et U est CZ₆,
   - R est H, alkyle, aralkyle, aryle, alcanoïque ou alkylsulfonique
   - Z₁, Z₂, Z₃, Z₄ et Z₅, sont indépendamment H, Br, Cl, F, I, alkyle, aryle, alkoxy, perfluoroalkyle, nitro, cyano, carboxyle, sulfonyle, incluant les esters ou amides de carboxyle ou sulfonyle
   et leurs sels.
b) ensemencer le milieu avec un échantillon biologique à tester,
c) laisser incuber, et
d) révéler la présence d'au moins une activité peptidase

Lorsque ledit milieu est un milieu pour détecter uniquement une seule variation de pH, Y₁ est H ou un alkyl

Lorsque ledit milieu est un milieu pour détecter une activité peptidase, Y₁ est un peptide. Préférentiellement U ou V est N.

Lorsque ledit milieu est un milieu pour détecter une activité peptidase, et une variation de pH, Y₁ est un peptide. Préférentiellement U ou V est N.

Comme indiqué précédemment, l'ensemencement des microorganismes peut être réalisé par toutes les techniques d'ensemencement connues de l'homme du métier. Une étape d'incubation peut être réalisée à une température pour laquelle l'activité enzymatique que l'on souhaite détecter est optimale, que l'homme du métier peut choisir aisément selon l'activité enzymatique à détecter. L'étape d) peut s'effectuer par un examen visuel, par colorimétrie ou fluorimétrie. Lors de l'étape d), on peut révéler la présence de l'activité peptidase, seule ou en combinaison avec d'autres activités enzymatiques. Dans certains cas, il peut être avantageux d'effectuer l'étape d) en présence d'un acide, tel que l'acide acétique.

Selon un mode préféré de réalisation, Y₁ est un peptide, préférentiellement choisi parmi l'alanine, l'asparagine, la glutamine, la tyrosine Selon un mode préféré de réalisation, n = 1

Selon un mode préféré de réalisation, W₁, W₂, W₃ et W₄ sont indépendamment H

Selon un mode préféré de réalisation, U est CH et V est N ou N⁺R, préférentiellement N⁺CH3

Selon un mode préféré de réalisation, V est CH et U est N ou N⁺R, préférentiellement N⁺CH3

Selon un mode préféré de réalisation, Z₁, Z₂, Z₃ et Z₄, Z₅, et Z₆ sont indépendamment H

Selon un mode préféré de réalisation, L-Alanyl-4-(4'-amidophenyl)-2-méthyl-quinoline, L-Alanyl-4-(4'-amidostyryl)-N-benzyl-quinolinium TFA, L-Glutamyl-4-(4'-amidostyryl)-N-benzyl-quinolinium TFA, beta-Alanyl-4-(4'-amidostyryl)-N-benzyl-quinolinium TFA, L-Alanyl-4-(4'-amidostyryl)-N-methylquinolinium TFA, D-Alanine-N-méthyl-4-(4'-aminostyryl)-quinolinium (dichlorure), L-Alanyl-2-(4'-amidostyryl)-N-méthyl-quinolinium, Z-Alanyl-2-(4'-amidostyryl)-N-méthyl-quinolinium, L-Alanyl-4-(4'-amidostyryl)-N-méthyl-quinolinium (dichlorure), L-Alanyl-L-alanyl-4-(4'-amidostyryl)-N-méthyl-quinolinium (dichlorure), L-Alanyl-L-alanyl-L-alanyl-4-(4'-amidostyryl)-N-méthyl-quinolinium (dichlorure), γ-Aminobutyryl-4-(4'-amidostyryl)-N-methylquinolinium di-Cl, L-Asparaginyl-4-(4'-amidostyryl)-N-méthyl-quinolinium-bis-TFA, α-Aspartyl-4-(4'-amidostyryl)-N-methylquinolinium dichloride, β-Aspartyl-4-(4'-amidostyryl)-N-methylquinolinium dichloride, Glycyl-4-(4'-amidostyryl)-N-méthyl-quinolinium (dichlorure), L-gamma-Glutamyl-4-(4'-amidostyryl)-N-méthyl-quinolinium (dichlorure), Glycyl-4-(4'-amidostyryl)-N-méthyl-quinolinium chloride, L-Leucyl-4-(4'-amidostyryl)-N-méthyl-quinolinium dihydrochloride, L-Methionyl-4-(4'-amidostyryl)-N-methyl-quinolinium di-Cl, Pyroglutamyl-4-(4'-amidostyryl)-N-méthyl-quinolinium iodide, Sarcosinyl-4-(4'-amidostyryl)-N-methyl-quinolinium dichloride, L-Tryptophanyl-4-(4-amidostyryl)-N-méthyl-quinolinium dihydrochloride, L-Tyrosyl-4-(4'-amidostyryl)-N-méthyl-quinolinium dihydrochloride, beta-Alanyl-2-(4'-amidostyryl)-N-methyl-quinolinium TFA, L-Prolyl-2-(4'-amidostyryl)-N-méthyl-quinolinium (HBr), Z-Arginyl-2-(4'-amidostyryl)-N-méthyl-quinolinium, L-Pyroglutamyl -2-(4'-amidostyryl)-N-méthyl-quinolinium, L-Alanyl-4-(4'-amidostyryl)quinoline TFA, β-Alanyl-4-(4'-amidostyryl)-quinoline, L-Glutamyl-4-(4'-amidostyryl)quinoline TFA, Glycyl-4-(4'-amidostyryl)quinoline TFA, Pyroglutamyl-4-(4'-amidostyryl) quinoline, L-Alanyl-2-(4'-amidostyryl)-quinoline, β-Alanyl-2-(4'-amidostyryl)-quinoline, Glycyl-2-(4'-amidostyryl)quinoline TFA, L-Alanyl-2-(2'-pyridyl)-4-(4"-amidostyryl)-quinoline Selon un mode préféré de réalisation de l'invention, ledit composé est choisi parmi : β-Alanyl-2-(4'-amidostyryl)-quinoline (β-Ala-ASQ), L-Tyrosyl-4-(4'-amidostyryl)-N-méthyl-quinolinium dihydrochloride (Tyr-ASQM⁺), L-Asparaginyl-4-(4'-amidostyryl)-N-méthyl-quinolinium-bis-TFA (Asn-ASQM⁺), L-gamma-Glutamyl-4-(4'-amidostyryl)-N-méthyl-quinolinium (dichlorure) (γ-Glu-ASQM⁺), L-Alanyl-L-alanyl-L-alanyl-4-(4'-amidostyryl)-N-méthyl-quinolinium (dichlorure) (Tri-Ala-ASQM⁺)

Préférentiellement, le microorganismes est choisi parmi *Escherichia coli, Serratia marcescens, Enterobacter cloacae, Salmonella typhimurium, Providencia rettgeri, Yersinia enterocolitica, Pseudomonas aeruginosa, Bacillus subtilis, Listeria monocytogenes, Staphylococcus aureus, Streptococcus pyogenes, Enterococcus faecalis.*

L'invention concerne également un milieu de détection et/ou d'identification de microorganismes comprenant un composé de la formule (I) suivante pour détecter une activité peptidase et/ou une variation de pH : 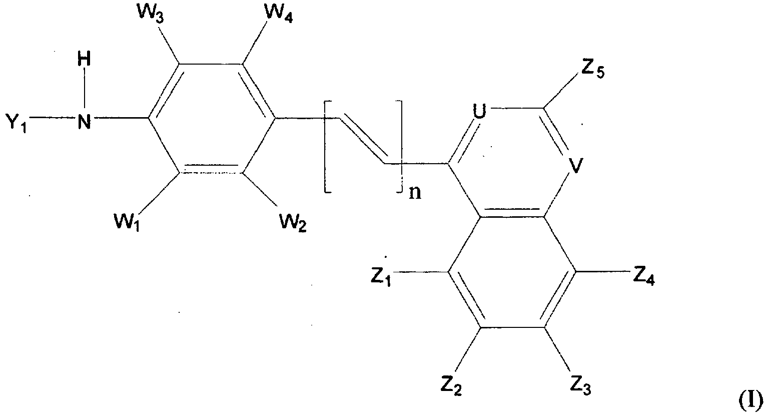 selon laquelle :
- Y₁ est un peptide, H ou un alkyl
- W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci
- n = 0, 1 ou 2
- U est N ou N⁺R et V est CZ₆ N ou N⁺R ou bien V est N ou N⁺R et U est CZ₆,
- R est H, alkyle, aralkyle, aryle, alcanoïque ou alkylsulfonique
- Z₁, Z₂, Z₃, Z₄ et Z₅, sont indépendamment H, Br, Cl, F, I, alkyle, aryle, alkoxy, perfluoroalkyle, nitro, cyano, carboxyle, sulfonyle, incluant les esters ou amides de carboxyle ou sulfonyle
   et leurs sels.

Lorsque ledit milieu est un milieu pour détecter une seule variation de pH , Y₁ est H ou un alkyl

Lorsque ledit milieu est un milieu pour détecter une activité peptidase, et facultativement d'une variation de pH, Y₁ est un peptide. Préférentiellement U ou V est N

Selon un mode préféré de réalisation de l'invention, Y₁ est un peptide, préférentiellement choisi parmi l'alanine, l'asparagine, la glutamine, la tyrosine Selon un mode préféré de réalisation de l'invention, n = 1

Selon un mode préféré de réalisation de l'invention, W₁, W₂, W₃ et W₄ sont indépendamment H

Selon un mode préféré de réalisation de l'invention, U est CH et V est N ou N⁺R, préférentiellement N⁺CH3

Selon un mode préféré de réalisation de l'invention, V est CH et U est N ou N⁺R, préférentiellement N⁺CH3

Selon un mode préféré de réalisation de l'invention, Z₁, Z₂, Z₃ et Z₄, Z₅, et Z₆ sont indépendamment H

Selon un mode préféré de réalisation de l'invention, L-Alanyl-4-(4'-amidophenyl)-2-méthyl-quinoline, L-Alanyl-4-(4'-amidostyryl)-N-benzyl-quinolinium TFA, L-Glutamyl-4-(4'-amidostyryl)-N-benzyl-quinolinium TFA, beta-Alanyl-4-(4'-amidostyryl)-N-benzyl-quinolinium TFA, L-Alanyl-4-(4'-amidostyryl)-N-methyl-quinolinium TFA, D-Alanine-N-méthyl-4-(4'-aminostyryl)-quinolinium (dichlorure), L-Alanyl-2-(4'-amidostyryl)-N-méthyl-quinolinium, Z-Alanyl-2-(4'-amidostyryl)-N-méthyl-quinolinium, L-Alanyl-4-(4'-amidostyryl)-N-méthyl-quinolinium (dichlorure), L-Alanyl-L-alanyl-4-(4'-amidostyryl)-N-méthyl-quinolinium (dichlorure), L-Alanyl-L-alanyl-L-alanyl-4-(4'-amidostyryl)-N-méthyl-quinolinium (dichlorure), γ-Aminobutyryl-4-(4'-amidostyryl)-N-methylquinolinium di-Cl, L-Asparaginyl-4-(4'-amidostyryl)-N-méthyl-quinolinium-bis-TFA, α-Aspartyl-4-(4'-amidostyryl)-N-methylquinolinium dichloride, β-Aspartyl-4-(4'-amidostyryl)-N-methylquinolinium dichloride, Glycyl-4-(4'-amidostyryl)-N-méthyl-quinolinium (dichlorure), L-gamma-Glutamyl-4-(4'-amidostyryl)-N-méthyl-quinolinium (dichlorure), Glycyl-4-(4'-amidostyryl)-N-méthyl-quinolinium chloride, L-Leucyl-4-(4'-amidostyryl)-N-méthyl-quinolinium dihydrochloride, L-Methionyl-4-(4'-amidostyryl)-N-methyl-quinolinium di-Cl, Pyroglutamyl-4-(4'-amidostyryl)-N-méthyl-quinolinium iodide, Sarcosinyl-4-(4'-amidostyryl)-N-methyl-quinolinium dichloride, L-Tryptophanyl-4-(4-amidostyryl)-N-méthyl-quinolinium dihydrochloride, L-Tyrosyl-4-(4'-amidostyryl)-N-méthyl-quinolinium dihydrochloride, beta-Alanyl-2-(4'-amidostyryl)-N-methyl-quinolinium TFA, L-Prolyl-2-(4'-amidostyryl)-N-méthyl-quinolinium (HBr), Z-Arginyl-2-(4'-amidostyryl)-N-méthyl-quinolinium, L-Pyroglutamyl -2-(4'-amidostyryl)-N-méthyl-quinolinium, L-Alanyl-4-(4'-amidostyryl)quinoline TFA, β-Alanyl-4-(4'-amidostyryl)-quinoline, L-Glutamyl-4-(4'-amidostyryl)quinoline TFA, Glycyl-4-(4'-amidostyryl)quinoline TFA, Pyroglutamyl-4-(4'-amidostyryl) quinoline, L-Alanyl-2-(4'-amidostyryl)-quinoline, β-Alanyl-2-(4'-amidostyryl)-quinoline, Glycyl-2-(4'-amidostyryl)quinoline TFA, L-Alanyl-2-(2'-pyridyl)-4-(4"-amidostyryl)-quinoline Selon un mode préféré de réalisation de l'invention, ledit composé est choisi parmi : β-Alanyl-2-(4'-amidostyryl)-quinoline (β-Ala-ASQ), L-Tyrosyl-4-(4'-amidostyryl)-N-méthyl-quinolinium dihydrochloride (Tyr-ASQM⁺), L-Asparaginyl-4-(4'-amidostyryl)-N-méthyl-quinolinium-bis-TFA (Asn-ASQM⁺), L-gamma-Glutamyl-4-(4'-amidostyryl)-N-méthyl-quinolinium (bichlorure) (γ-Glu-ASQM⁺), L-Alanyl-L-alanyl-L-alanyl-4-(4'-amidostyryl)-N-méthyl-quinolinium (dichlorure) (Tri-Ala-ASQM⁺)

Préférentiellement, le microorganismes est choisi parmi *Escherichia coli, Serratia marcescens, Enterobacter cloacae, Salmonella typhimurium, Providencia rettgeri, Yersinia enterocolitica, Pseudomonas aeruginosa, Bacillus subtilis, Listeria monocytogenes, Staphylococcus aureus, Streptococcus pyogenes, Enterococcus faecalis.*

Préférentiellement, ledit milieu réactionnel est un milieu de détection et/ou d'identification de microorganismes, ledit milieu comprenant au moins une molécule utilisée à titre de substrat enzymatique ou d'indicateur de pH telle que définie ci avant. Préférentiellement, ledit composé, substrat enzymatique ou indicateur de pH, est à une concentration comprise entre 1 et 1000 mg/l, préférentiellement entre 10 et 500 mg/l. Selon un mode particulier de réalisation de l'invention, ledit milieu de détection et/ou d'identification selon l'invention comprend en outre au moins un autre substrat enzymatique, spécifique d'une activité enzymatique différente de l'activité peptidase détectée par la molécule selon l'invention.

Selon un autre mode particulier de réalisation de l'invention, ledit milieu de détection et/ou d'identification selon l'invention comprend en outre au moins un substrat spécifique d'une activité enzymatique différente de celle mise en évidence par la variation de pH.

Le métabolisme enzymatique du ou des autres substrats génère un signal détectable, différent du signal généré par le composé selon l'invention utilisé à titre de substrat enzymatique ou d'indicateur de pH, comme par exemple des produits colorés ou fluorescents différents, pour permettre la mise en évidence comme la détection et/ou l'identification et/ou la quantification d'un ou plusieurs microorganismes. A titre d'autre substrat spécifique, on peut utiliser tout autre substrat classiquement utilisé dans la détection des microorganismes. La concentration de l'autre substrat enzymatique spécifique est généralement comprise entre 0,01 et 1 g/l. L'homme du métier pourra déterminer facilement une telle concentration en fonction du substrat utilisé. A titre indicatif, il est possible de combiner les composés selon l'invention avec des substrats enzymatiques de peptidase, d'osidase, d'estérase ou de réductase. En particulier, il est possible d'associer un substrat selon l'invention pour lequel le peptide est une β-Alanine avec un substrat d'osidase, tel que le 5-Bromo-4-chloro-3-indolyl-β-glucoside, ou l'Alizarine-β-galactoside. Il est également possible d'associer un substrat selon l'invention pour lequel le peptide est la L-Alanine avec un substrat d'estérase, tel que le 5-Bromo-6-chloro-3-indoxyl-octanoate ou le 5-Bromo-3-indoxyl-phosphate.

Selon un mode particulier de réalisation de l'invention, ledit milieu de détection et/ou d'identification selon l'invention comprend en outre au moins un autre substrat enzymatique spécifique de l'activité peptidase ou spécifique de l'activité enzymatique détectée par un composé selon l'invention utilisé à titre d'indicateur de pH. Par le choix particulier de substrats, il est alors possible d'identifier des groupes de microorganismes exprimant une même activité enzymatique. La concentration de l'autre substrat enzymatique spécifique est généralement comprise entre 0,01 et 1 g/l. L'homme du métier pourra déterminer facilement une telle concentration en fonction du substrat utilisé. En particulier, il est possible d'associer un substrat selon l'invention pour lequel le peptide est une L-Alanine avec un substrat de L-Alanine aminopeptidase décrit dans la demande WO2006030119, tel que la L-Alanine-pentyl-résorufamine. Selon un mode particulier de réalisation de l'invention, ledit milieu de détection et/ou d'identification selon l'invention comprend en outre au moins un indicateur métabolique, spécifique d'une activité métabolique différente de celle détectée par le composé selon l'invention utilisé à titre de substrat ou d'indicateur de pH.

Cet indicateur métabolique peut notamment être une source de Carbone ou d'Azote associée ou non à un réactif révélant son métabolisme. Selon un mode particulier, la source de Carbone ou d'Azote est associée à un indicateur de pH différent du composé selon l'invention utilisé à ce titre. Selon un autre mode particulier, la source de Carbone ou d'Azote est associée à un cation. Selon un autre mode particulier l'indicateur métabolique permet de détecter une activité tryptophanase et associe du Tryptophane et un réactif permettant de détecter la production d'Indole.

Les exemples ci après sont donnés a titre illustratif.

### Exemple 1 - Synthèse de substrats

La 4-(4'-Aminostyryl)-quinoléine était préparé suivant la méthode de Bahner *et al* (C. T. Bahner, C. Cook, J. Dale, J. Fain, F. Hannan, P. Smith and J. Wilson, Journal of Organic Chemistry, 1958, 23, 1060-1062).

L'aminoacylation de la 4-(4'-aminostyryl)-quinoléine avec de la t-BOC-β-alanine est donnée à titre d'exemple et les autres aminoacylations étaient effectuées de la même façon avec un autre acide aminé protégé.

Synthèse de la t-BOC-β-alanine-4-(4'-aminostyryl)-quinoléine à partir de la 4-(4'-aminostyryl)-quinoléine et de la t-BOC-β-alanine suivant la méthode des anhydrides mixés :
On s'est assuré que toute la verrerie utilisée était bien sèche avant toute chose car la présence d'humidité aurait pu empêcher la réaction de se dérouler correctement. On a dissous 0,50g (0,00266 mol) de t-Boc-L-alanine dans 10ml de tétrahydrofuranne (THF) sec et ajouté un piège de CaCl₂ sur le col du ballon afin d'assurer l'étanchéité. On a agité magnétiquement et fait descendre la température du milieu réactionnel à -15°C en utilisant un bain de glace congelée mélangée à des sels de NaCl. Une fois cette température atteinte, on a laissé agiter 5 minutes de plus. Puis on a ajouté 0,34g (0,0033 mol) de N-Méthylmorpholine tout en prenant bien soin que la température redescende à -15 °C avant de procéder à la prochaine étape. Ensuite on a ajouté goutte à goutte avec la plus grande précaution 0,36g (0,00266 mol) d'Isochlorobutylformate, le milieu réactionnel s'est troublé comme prévu à chaque goutte ajoutée. Quelques minutes plus tard on a dissous 0,49g (0,002 mol) de 4-(4'-aminostyryl)-quinoléine dans le minimum de THF sec et on l'a ajouté au milieu réactionnel. On a retiré le bain de glace après 2 heures et laissé agiter durant la nuit. On a filtré la solution, rincé le ballon avec du THF normal et conservé le filtrat. Un léger précipité fut présent on est donc passé à la prochaine étape, autrement on aurait du remettre la solution dans un ballon propre et évaporer la quantité adéquate de solvant. On a transféré la solution à l'aide d'une pipette pasteur dans un bécher de 400 ml contenant de la glace, de l'eau ainsi que du carbonate de sodium (Na₂CO₃). On a laissé le tout agiter durant une paire d'heure, jusqu'à obtenir une solution limpide. On a filtré la solution dans un petit Buchner puis dissous le précipité dans de l'éthanol afin de le recristalliser. On a ainsi obtenu 0,76g de t-BOC-β-alanine-4-(4'-aminostyryl)-quinoléine, un solide jaune avec un rendement de 90%, m.p. 160-162 °C. HRMS (EI) pour C₂₅H₂₅N₃O₃. Masse théorique de l'ion moléculaire 418,2125 (M+H)⁺. Masse trouvée: 418,2123. ¹H-NMR: (DMSO) δ 1.34 (9H, s, C(C*H*₃)₃), 3.19 (2H, q, J=5.94 Hz, >C*H*₂), 3.38 (2H, m, >C*H*₂), 6.88 (1H, t, J=5.69 Hz, N*H*), 7.50 (1H, d, J=16.08 Hz, =C*H*), 7.61 (1H, m, Ph-*H*), 7.64 (2H, d, J=8.66 Hz, C6'-H and C2'-*H*), 7.73 (1H, m, Ph-*H*), 7.74 (2H, d, J=8.91 Hz, C5'-*H* and C3'-*H*), 7.79 (1H, d, J=4.70 Hz, C3-*H*), 7.96 (1H, d, J=6.33 Hz, =C*H*), 7.99 (1H, d, J=8.16 Hz, C5-*H*), 8.48 (1H, d, J=8.41 Hz, C8-*H*), 8.82 (1H, d, J=4.70 Hz, C2-*H*), 10.09 (1H, s, N*H*). IR: υₘₐₓ cm⁻¹ 3380 (CO), 3307 (CONH), 1693 (CONH), 1673 (CONH), 1365 (C(CH₃)₃), 754 (CH₂).

La déprotection des composés t-BOC-acide aminé était accomplie soit par réaction avec de l'HCl (donnant un sel d'acide chlorhydrique) soit par réaction avec de l'acide TFA (donnant un sel d'acide trifluoroacétique). La déprotection de la t-BOC-β-alanine-4-(4'-aminostyryl)-quinoléine avec de l'HCl est donnée à titre d'exemple et les autres déprotections étaient effectuées de la même façon. Pour la déprotection avec TFA, on a placé du composé à déprotéger dans un ballon équipé d'un piège à CaCl₂. Puis on a ajouté de l'acide trifluoroacétique et laissé le mélange sous agitation magnétique toute la nuit. On a évaporé le TFA afin d'obtenir le produit sous forme de sel d'acide trifluoroacétique.

Synthèse de la β-Alanine-4-(4'-aminostyryl)-quinoléine à partir de la t-BOC-β-alanine-4-(4'-aminostyryl)-quinoléine :
On a dissout de la t-BOC-β-alanine-4-(4'-aminostyryl)-quinoléine dans le minimum d'acétate d'éthyle dans un ballon équipé d'un piège à CaCl₂. Puis, on a ajouté une solution saturée d'HCl dans de l'acétate d'éthyle. On a laissé agiter le mélange pendant la nuit et ensuite on a évaporé le solvant. On a ainsi obtenu de la β-Alanine-4-(4'-aminostyryl)-quinoléine dichlorique, un solide marron avec un rendement de 77%, m.p. 176-178 °C. HRMS (EI) for C₂₀H₁₉N₃O. Masse théorique de l'ion moléculaire 318,1601 (M+H)⁺. Masse trouvée: 318,1601. ¹H-NMR: (DMSO) δ 2.50 (2H, t, J=6.19 Hz, >C*H*₂), 3.15 (2H, m, >C*H*₂), 7.27 (1H, d, J=2.72 Hz, Ph-*H*), 7.30 (1H, d, J=16.08 Hz, =C*H*), 7.57 (2H, d, J=8.41 Hz, Ph-*H*), 7.63 (1H, d, J=16.08 Hz, -C*H*), 7.64 (2H, d, J=8.91 Hz, Ph-*H*), 7.74 (2H, m, Ph-H*)*, 8.12 (1H, d, J=8.66 Hz, Ph-*H*) 8.23 (1H, d, J=7.42 Hz, C8-*H*), 8.88 (1H, d, J=4.45 Hz, C2-*H*), 10.43 (1H, s, N*H*). IR: υₘₐₓ cm⁻¹ 2852 (NH salt), 1674 (C=O), 1584 (NH), 1379 (NH).

L'alkylation des composés t-BOC-acide aminé était effectuée par réaction avec un alkyle halogéné. Les sels que l'on a ainsi obtenus étaient déprotégés soit avec de l'HCl soit avec de l'acide TFA comme mentionné ci-dessus. La méthylation et la déprotection de la t-BOC-β-alanine-4-(4'-aminostyryl)-quinoléine avec de l'iodure de méthyle est donnée à titre d'exemple.

Synthèse de la β-Alanine-N-méthyl-4-(4'-aminostyryl)-quinoléinium dichlorée à partir de la t-BOC-β-alanine-4-(4'-aminostyryl)-quinoléine :
On a agité un mélange de 0,42g (0,001 mols) de la t-Boc-β-alanyl-4-(4'-aminostyryl)-quinoléine et de 2,21g (0,0155 mols) d'iodure de méthyle dans 10 ml d'acétonitrile pendant la nuit. On a filtré le mélange donnant 0,38g d'un solide orange de t-Boc-β-alanyl-N-méthyl-4-(4'-aminostyryl)-quinoléinium iodé avec un rendement de 70%, 180-182 °C, HRMS (EI) for C₂₆H₂₉N₃O₃. Masse théorique de l'ion moléculaire 432,2282 (M+H)⁺. Masse trouvée: 432,2280. ¹H-NMR: (DMSO) δ 1.39 (9H, s, - C(C*H*₃)₃), 3.24 (2H, quartet, J= 6.19 Hz, >C*H*₂), 3.37 (2H, m, >C*H*₂), 4.54 (3H, s, - C*H*₃), 6.95 (1H, t, J=6.19 Hz, N*H*), 7.75 (2H, d, J=8.66 Hz, C6'-*H* and C2'-*H*), 7.96 (2H, d, J=8.66 Hz, C5'-*H* and C3'-*H*), 8.05 (1H, t, J=7.42 Hz, C7-*H*), 8.08 (1H, d, J=16.08 Hz, =C*H*), 8.24 (1H, d, J=16.08 Hz, =C*H*), 8.27 (1H, t, J=6.93 Hz, C6-*H*), 8.44 (1H, d, J=8.91 Hz, C5-*H*), 8.48 (1H, d, J=6.68 Hz, C3-*H*), 9.05 (1H, d, J=8.66 Hz, C8-*H*), 9.32 (1H, d, J=6.68 Hz, C2-*H*), 10.26 (1H, s, N*H*). IR: υₘₐₓ cm⁻¹ 3368 (NH), 3276 (NH), 1674 (CO), 1617 (CO), 1592 (CONH), 1571 (CON*H*), 1365 (CCH₃)₃), 1395 (CH₃).

On a déprotégé 0,54g (0,0013 mols) de ce composé avec de l'HCl donnant 0,39g d'un solide marron de β-Alanine-N-méthyl-4-(4'-aminostyryl)-quinoléinium dichlorée avec un rendement de 77%, m.p. 176-178 °C. HRMS (EI) for C₂₀H₁₉)N₃O. Masse théorique de l'ion moléculaire 318,1601 (M+H)⁺. Masse trouvée: 318,1601. ¹H-NMR: (DMSO) δ 2.50 (2H, t, J=6.19 Hz, >C*H*₂), 3.15 (2H, m, >C*H*₂), 7.27 (1H, d, J=2.72 Hz, Ph-*H*), 7.30 (1H, d, J=16.08 Hz, =C*H*), 7.57 (2H, d, J=8.41 Hz, Ph-*H*), 7.63 (1H, d, J=16.08 Hz, -C*H*), 7.64 (2H, d, J=8.91 Hz, Ph-*H*), 7.74 (2H, m, Ph-*H*), 8.12 (1H, d, J=8.66 Hz, Ph-*H*), 8.23 (1H, d, J=7.42 Hz, C8-*H*), 8.88 (1H, d, J=4.45 Hz, C2-*H*), 10.43 (1H, s, N*H*). IR: υₘₐₓ cm⁻¹ 2852 (NH salt), 1674 ( C=O), 1584 (NH), 1379 (NH).

Autre exemples de substrats typiques obtenus d'une façon similaire à celle mentionné ci-dessus :
L-Alanine-N-méthyl-4-(4'-aminostyryl)-quinoléinium dichlorée, un solide rouge, m.p. 244-246 °C. HRMS(EI) for C₂₁H₂₃N₃O. Masse théorique de l'ion moléculaire 332,1757 (M-2HCl+H)⁺. Masse trouvée: 332,1756. ¹H-NMR: (DMSO) δ 1.51 (3H, d, J=6.93 Hz, -C*H*₃), 4.19 (1H, q, J=6.93 Hz, ala-*H*), 4.55 (3H, s, -C*H*₃), 7.86 (2H, d, J=8.66 Hz, C6'-*H* and C2'-*H*), 8.01 (2H, d, J=8.66 Hz, C5'-*H* and C3'-*H*), 8.05 (1H, m, Ph-*H*), 8.28 (1 H, m, Ph-*H*), 8.16 (1H, d, J=16.08 Hz, =C*H*), 8.29 (1H, d, J=16.08 Hz, =C*H*), 8.44 (1H, d, J=8.91 Hz, C5-*H*), 8.51 (1H, d, J=6.68 Hz, C3-*H*), 9.06 (1H, d, J=8.41 Hz, C8-*H*), 9.40 (1H, d, J=6.68 Hz, C2-*H*), 11.49 (1*H*, s, N*H*). IR: υₘₐₓ cm⁻¹ 1693 (C=O), 1597 (NH), 1533 (NH), 1324 (NH), 1369 (CH₃).
L-gamma-Glutamyl-N-méthyl-4-(4'-aminostyryl)-quinoléinium dichlorée.
   ¹H-NMR: (DMSO) δ 2.05 (2H, m, >C*H*₂), 2.55 (2H, m, >C*H*₂), 3.87 (1H, m, >C*H*-), 4.49 (3H, s, N-C*H*₃), 7.70 (2H, d, J=7 Hz, Ar-*H*), 7.90 (2H, d, J=7 Hz, Ar-*H*), 7.90-8.50 (6H, m, Ar-*H*, -CH=), 8.60 (1H, broad s, >N*H*), 8.97 (1H, d, J=8 Hz, Ar-*H*), 9.29 (1H, d, J=5 Hz, Ar-*H*).
L-Tyrosyl-N-méthyl-4-(4'-aminostyryl)-quinoléinium dichlorée.
   ¹H-NMR: (DMSO) δ 3.10 (2H, m, >C*H*₂), 4.30 (1H, m, >C*H*-), 4.52 (3H, s, N-C*H*₃), 6.70 (2H, d, J=7 Hz, Ar-*H*), 7.12 (2H, d, J=7 Hz, Ar-*H*), 7.79 (2H, d, J=7 Hz, Ar-*H*), 7.98 (2H, d, J=7Hz, Ar-*H*), 8.00-8.60 (6H, m, Ar-*H* and -C*H*=), 9.04 (1H, d, J=7 Hz, Ar-*H*), 9.38 (1H, d, J=5 Hz, Ar-*H*), 9.46 (1H, broad s, >O*H*).
L-Alanine-N-méthyl-4-(4'-aminostyryl)-quinoléinium dichlorée et D-Alanine-N-méthyl-4-(4'-aminostyryl)-quinoléinium dichlorée.
   ¹H-NMR: (DMSO) δ 1.51 (3H, d, J=6.93 Hz, -C*H*₃), 4.19 (1H, q, J=6.93 Hz, ala-*H*), 4.55 (3H, s, -C*H*₃), 7.86 (2H, d, J=8.66 Hz, C6'-*H* and C2'-*H*), 8.01 (2H, d, J=8.66 Hz, C5'-*H* and C3'-*H*), 8.05 (1H, m, Ph-*H*), 8.28 (1H, m, Ph-*H*), 8.16 (1H, d, J=16.08 Hz, =C*H*), 8.29 (1H, d, J=16.08 Hz, =C*H*), 8.44 (1H, d, J=8.91 Hz, C5-*H*), 8.51 (1H, d, J=6.68 Hz, C3-*H*), 9.06 (1H, d, J=8.41 Hz, C8-*H*), 9.40 (1H, d, J=6.68 Hz, C2-*H*), 11.49 (1H, s, N*H*).

### Exemple 2 - Utilisation de substrats de formule 1 selon l'invention pour détecter une activité peptidase

### a) Substrats de peptidase

Les composés ß-Alanyl-2-(4'-amidostyryl)-quinoline (β-Ala-ASQ), L-Tyrosyl-4-(4'-amidostyryl)-N-méthyl-quinolinium dihydrochloride (Tyr-ASQM⁺), L-Asparaginyl-4-(4'-amidostyryl)-N-méthyl-quinolinium-bis-TFA (Asn-ASQM⁺), L-gamma-Glutamyl-4-(4'-amidostyryl)-N-méthyl-quinolinium (dichlorure) (γ-Glu-ASQM⁺), L-Alanyl-L-alanyl-L-alanyl-4-(4'-amidostyryl)-N-méthyl-quinolinium (dichlorure) (Tri-Ala-ASQM⁺), ont été synthétisés comme décrit à l'exemple 1

### b) Préparation du milieu

40mg de chacun des substrats ont été dissouts dans 4ml de Diméthylsulfoxide (80mg pour le Asn-ASQM⁺) et ajouté à 400ml de milieu Columbia préalablement autoclavé. Les 6 milieux ont été répartis en boîte de Petri de 90mm de diamètre à raison de 20ml par boîte.

### c) Ensemencement et incubation

Dix-sept souches de micro-organismes issues de collections et appartenant à différentes espèces de bactéries et levures sont ensemencées en spot d'environ 10 000 unités formant colonies.

Dix microlitres d'un mélange à parts égales de suspensions à 0,5 McFarland diluées au 20^{e} des souches *Escherichia coli* NCTC 10 418 et *Yersinia enterocolitica* NCTC 11 176 sont ensemencés sur le milieu par isolement en quadrants.

Les milieux sont incubés 24 heures à 37°C, puis les colonies formées sont examinées visuellement, avec ou sans ajout d'acide, et sous lampe UV à 365nm.

### d) Lecture des résultats

Les résultats obtenus sont présentés dans le tableau 1 ci après

| **Espèce** | **Tyr-ASQM⁺** | | **Tri-Ala-ASQM⁺** | | **γ-Glu-ASQM⁺** | | **Asn-ASQM⁺** | | **β-Ala-ASQ** | | | |
|---|---|---|---|---|---|---|---|---|---|---|---|---|
| **Numéro de collection** | **Crois.** | **Coul.** | **Crois.** | **Coul.** | **Crois.** | **Coul.** | **Crois.** | **Coul.** | **Crois.** | **Fluo.** | **Coul.** | **Coul. post acid.** |
| *Escherichia coli* NCTC 10 418 | 2 | Orange | 2 | Orange | 2 | Orange | 2 | Orange pâle | 2 | Bleu pâle | | Jaune Pâle |
| *Serratia marcescens* NCTC 10 211 | 2 | Orange | 2 | Orange | 2 | Orange | 2 | Orange pâle | - 2 | Vert | | Orange |
| *Pseudomonas aeruginosa* NCTC 10 662 | 2 | - | 2 | Orange | 2 | Orange | 2 | Orange pâle | 2 | Vert | | Orange |
| *Yersinia enterocolitica* NCTC 11 176 | 2 | Orange pâle | 2 | Orange pâle | 2 | - | 2 | - | 2 | Bleu pâle | | Jaune Pâle |
| *Salmonella typhimurium* NCTC 74 | 2 | Orange pâle | 2 | Orange | 2 | Orange | 2 | - | 2 | Bleu pâle | | Jaune Pâle |
| *Enterobacter cloacae* NCTC 11 936 | 2 | Orange | 2 | Orange | 2 | Orange | 2 | Orange pâle | 2 | Bleu pâle | | Jaune Pâle |
| *Providencia rettgeri* NCTC 7 475 | 2 | Orange pâle | 2 | Orange pâle | 2 | Orange | 2 | - | 2 | Bleu pâle | | Jaune Pâle |
| *Bacillus subtilis* NCTC 9 372 | 1 | - | 2 | - | 1 | - | 2 | Orange | PdC | - | - | - |
| *Enterococcus faecalis* NCTC 775 | 1 | Orange pâle | 2 | Orange pâle | 1 | - | 2 | - | PdC | - | - | - |
| *Enterococcus faecium* NCTC 7 171 | 1 | - | 2 | - | 1 | - | 2 | - | PdC | - | - | - |
| *Staphylococcus epidermidis* NCTC 11 047 | 0,5 | - | 2 | - | 1 | - | - | - | PdC | - | - | - |
| *Staphylococcus aureus* NCTC 6 571 | PdC | - | 2 | - | 1 | - | 0,5 | - | PdC | - | - | - |
| *Staphylococcus aureus* NCTC 11 939 | PdC | - | 2 | - | 1 | - | 2 | Orange | PdC | - | - | - |
| *Streptococcus pyogenes* NCTC 8 306 | PdC | - | 2 | Orange pâle | 1 | - | - | - | PdC | - | - | - |
| *Listeria monocylogenes* NCTC 11 994 | 1 | - | 2 | - | 1 | - | 2 | - | PdC | - | - | - |
| *Candida albicans* ATCC 90 028 | 0,5 | - | 2 | - | 1 | - | 2 | - | PdC | - | - | - |
| *Candida glabrata* NCPF 3 943 | PdC | - | 2 | - | 1 | - | 0,5 | - | PdC | - | - | - |

| | | | | | | | | | | | | |
|---|---|---|---|---|---|---|---|---|---|---|---|---|
| PdC : pas de croissance, 0,5 : croissance faible, 1 : croissance modérée, 2 : bonne croissance, - : pas de couleur | | | | | | | | | | | | |

Sur la boîte ensemencée avec le mélange de 2 souches (*E. coli* et *Y. enterocolitica*), deux types de colonies se développent : des colonies orange de la souche d'*E*. *coli* et des colonies incolores de la souches d' *Y*. *enterocolitica.*

### e) Conclusions

Sur les milieux selon l'invention, il est possible de détecter des activités peptidase de micro-organismes grâce à la fluorescence ou à la coloration des colonies. Avec certains substrats selon l'invention, la coloration des colonies apparaît en condition acide, comme c'est le cas avec le β-Ala-ASQ dans cet exemple. Avec les autres substrats de cet exemple, l'étape d'acidification n'est pas requise.

Le plus souvent les substrats selon l'invention permettent la croissance de tout type de microorganismes: bactéries à Gram négatif, bactéries à Gram positif, levures, ..., mais certains peuvent être toxiques vis-à-vis de certains groupes, comme c'est le cas du β-Ala-ASQ dans cet exemple.

Par les différentes variations de structure des substrats de peptidase selon l'invention, il est possible de distinguer différents groupes de microorganismes. De plus, la coloration ne diffusant pas dans le milieu réactionnel, il est possible de distinguer et de compter les cellules ou colonies exprimant l'activité peptidase de celles ne l'exprimant pas.

### Exemple 3 - Utilisation de substrats de formule I selon l'invention pour détecter une activité peptidase

### a) Substrats de peptidase

Le composé pyroglutamyl-4-(4'-amidostyryl)-N-methylquinaldinium iodide a été synthétisé comme décrit à l'exemple 1

### b) Préparation du milieu

Une solution-mère à 50g/L en pyroglutamyl-4-(4'-amidostyryl)-N-methylquinaldinium iodide a été préparée dans un solvant type DMSO et ajoutée dans un milieu Columbia préalablement autoclavé et maintenu en surfusion, à raison de 75mg/L. Après homogénéisation, ce milieu a été réparti en boîtes carrées de 120 mm. Un milieu servant de témoin de croissance (Columbia) et noté T a également été coulé.

### c) Ensemencement et incubation

Vingt-deux souches de micro-organismes ont été ensemencées en spots-tests : dépôt de 1 µL à partir de suspensions bactériennes à 0.5McFarland (15.10⁴ CFU/spot)

Les milieux ont été incubés 48 heures à 37°C, puis les spots formés ont été examinés visuellement.

### d) Lecture des résultats

Les résultats obtenus sont présentés dans le tableau ci-après.

| | T | Pyroglutamyl-4-(4'-amidostyryl)-N-methylquinaldinium iodide | | |
|---|---|---|---|---|
| Nom souches *Ref. interne* | Croissance | Croissance | Intensité de coloration | couleur |
| *Escherichia coli 0002005* | +++ | +++ | - | |
| *Serratia marcescens 7303004* | +++ | +++ | ++ | Pigment naturel |
| *Pseudomonas aeruginosa 0001001* | +++ | +++ | +++ | Orange-rouge |
| *Burkholderia cepacia 7507032* | ++ | ++ | - | |
| *Yersinia enterocolitica 7605019* | ++ | + | - | |
| *Salmonella ser. Typhimurium 0801029* | +++ | +++ | - | |
| *Citrobacter freundii 0006197* | +++ | +++ | - | |
| *Morganella morganii 9207053* | +++ | +++ | - | |
| *Enterobacter cloacae 0604040* | +++ | +++ | - | |
| *Providencia rettgeri 0706064* | +++ | +++ | - | |
| *Klebsiella pneumoniae 0008009* | +++ | +++ | - | |
| *Acinetobacter baumannii 0604029* | +++ | +++ | - | |
| | | | | |
| *Bacillus subtilis 0204038* | +++ | - | - | |
| *Enterococcus faecalis 0008192* | ++ | ++ | - | |
| *Enterococcus faecium 0610021* | ++ | ++ | - | |
| *Staphylococcus epidermidis 0203050* | ++ | - | - | |
| *Staphylococcus aureus 7811049* | ++ | ++ | - | |
| *MRSA* | +++ | ++ | - | |
| *0303033* | | | | |
| *Streptococcus pyogenes 9806380* | - | - | - | |
| *Listeria monocytogenes 8812049* | ++ | ++ | - | |
| | | | | |
| *Candida albicans 0801025* | ++ | + | - | |
| *Candida glabrata 0408083* | + | - | - | |

| | | | | |
|---|---|---|---|---|
| Légende : +++ : bonne croissance/intensité de coloration élevée ++ : moyenne croissance/intensité de coloration moyenne + : faible croissance/intensité de coloration faible - : pas de croissance/pas de coloration | | | | |

### Exemple 4 - Utilisation de substrats de formule I selon l'invention pour détecter une activité peptidase

### a. Substrats de peptidase

Les composés L-alanyl-4-(4'-amidostyryl)-N-methylquinolinium (dichlorure), L-alanyl-L-alanyl-4-(4'-amidostyryl)-N-methylquinolinium (dichlorure), L-alanyl-L-alanyl-L-alanyl-4-(4'-amidostyryl)-N-methylquinolinium (dichlorure) ont été synthétisés comme décrit à l'exemple 1

### b. Préparation du milieu

Une solution-mère à 50g/L pour chacun des trois substrats a été préparée dans un solvant type DMSO et ajoutée dans un milieu Columbia préalablement autoclavé et maintenu en surfusion, à raison de 75mg/L. Après homogénéisation, les milieux ont été répartis en boîtes carrées de 120 mm. Un milieu servant de témoin de croissance (Columbia) et noté T a également été coulé.

### c. Ensemencement et incubation

Vingt-deux souches de micro-organismes ont été ensemencées en spots-tests: dépôt de 1 µL à partir de suspensions bactériennes à 0.5McFarland (15.10⁴ CFU/spot).

Les milieux ont été incubés 48 heures à 37°C, puis les spots formés ont été examinés visuellement.

### d. Résultats

Les résultats obtenus sont consignés dans le tableau ci-après.

| | T | L-alanyl-4-(4'-amidostyryl)-N methylquinolinium (dichlorure) | | | L-alanyl-L-alanyl-4-(4'-amidostyryl)-N methylquinolinium (dichlorure) | | | L-alanyl-L-alanyl-L-alanyl-4-(4'-amidostyryl)-N methylquinolinium (dichlorure) | | |
|---|---|---|---|---|---|---|---|---|---|---|
| Nom souches | Croissance | Croissance | Intensité de coloration | Couleur | Croissance | intensité de coloration | Couleur | Croissance | Intensité de coloration | Couleur |
| *Escherichia coli* | ++ | ++ | ++ | orange | ++ | - | - | ++ | ++ | orange |
| *Serratia marcescens* | ++ | ++ | ++ | orange | ++ | + | Orange | ++ | ++ | orange |
| *Pseudomonas aeruginosa* | ++ | ++ | ++ | orange | ++ | - | - | ++ | ++ | orange |
| *Burkholderia cepacia* | ++ | ++ | + | orange | ++ | - | - | ++ | + | orange |
| *Yersinia enterocolitica* | ++ | ++ | ++ | orange | ++ | - | - | ++ | ++ | orange |
| *Salmonelle ser typhimurium* | ++ | ++ | + | orange | ++ | - | - | ++ | + | orange |
| *Citrobacter freundii* | ++ | ++ | + | orange | ++ | - | - | ++ | + | orange |
| *Morganella morganü* | ++ | ++ | ++ | orange | ++ | - | - | ++ | ++ | orange |
| *Enterobacter cloacae* | ++ | ++ | ++ | orange | ++ | + | orange | ++ | ++ | orange |
| *Providencia rettgeri* | ++ | ++ | + | orange | ++ | - | - | ++ | + | orange |
| *Klebsiella pneumoniae* | ++ | ++ | ++ | orange | ++ | + | orange | ++ | ++ | orange |
| *Acinetobacter baumannii* | ++ | ++ | ++ | orange | ++ | + | orange | ++ | ++ | orange |
| | | | | | | | | | | |
| *Bacillus subtilis* | ++ | ++ | - | - | ++ | - | - | ++ | - | - |
| *Enterococcus faecalis* | ++ | ++ | + | orange | ++ | - | - | ++ | + | orange |
| *Enterococcus faecium* | ++ | ++ | - | - | ++ | - | - | ++ | - | - |
| *Staphylococcus epidermidis* | ++ | ++ | ++ | orange | ++ | - | - | ++ | ++ | orange |
| *Staphylococcus aureus* | ++ | ++ | + | jaune orange | ++ | - | - | ++ | + | jaune orange |
| *MRSA* | ++ | ++ | + | orange | ++ | - | - | ++ | + | orange |
| *Streptococcus pyogenes* | ++ | ++ | + | orange | ++ | + | orange | ++ | + | orange |
| *Listeria monocytogenes* | ++ | ++ | ++ | orange | ++ | + | orange | ++ | ++ | orange |
| | | | | | | | | | | |
| *Candida albicans* | ++ | ++ | - | | + | - | | + | | |
| *Candida glabrata* | + | + | - | | - | - | | - | | |

| | | | | | | | | | | |
|---|---|---|---|---|---|---|---|---|---|---|
| *Nota bene :* les références internes des souches sont le mêmes que dans l'exemple 3 Légende : ++ : moyenne croissance/intensité de coloration moyenne + : faible croissance/intensité de coloration faible - : pas de croissance/pas de coloration | | | | | | | | | | |

### e. Conclusion

Les trois substrats L-alanyl-4-(4'-amidostyryl)-N-methylquinolinium (dichlorure), L-alanyl-L-alanyl-4-(4'-amidostyryl)-N-methylquinolinium (dichlorure) et L-alanyl-L-alanyl-L-alanyl-4-(4'-amidostyryl)-N-methylquinolinium (dichlorure) permettent de détecter une activité peptidase. Le nombre d'acides aminés permet de jouer sur la spécificité de détection.

### Exemple 5 - Utilisation de substrats de formule I selon l'invention pour détecter une activité peptidase

### a) Substrats de peptidase

Les composés L-alanyl-4-(4'-amidostyryl)-N-methylquinolinium (dichlorure) et L-alanyl-2-(4'-amidostyryl)-N-methylquinolinium (dichlorure) ont été synthétisés comme décrit à l'exemple 1.

### b) Préparation du milieu

Une solution-mère à 50g/L pour chacun des deux substrats a été préparée dans un solvant type DMSO et ajoutée dans un milieu Columbia préalablement autoclavé et maintenu en surfusion, à raison de 75mg/L. Après homogénéisation, les milieux ont été répartis en boîtes de Petri de 55mm de diamètre. Un milieu servant de témoin de croissance (Columbia) et noté T a également été coulé.

### c) Ensemencement et incubation

Vingt-deux souches de micro-organismes ont été ensemencées à l'oëse calibrée de 10µL selon la méthode des trois cadrans à partir de suspensions bactériennes à 0.5McFarland. Les milieux ont été incubés 48 heures à 37°C, puis les colonies formées ont été examinées visuellement.

### d) Résultats

Les résultats obtenus sont consignés dans le tableau ci-après.

| | T | L-alanyl-4-(4'-amidostyryl)-N-methylquinolinium . (dichlorure) | | | L-alanyl-2-(4'-amidostyryl)-N-methylquinolinium (dichlorure) | | |
|---|---|---|---|---|---|---|---|
| Nom des souches | Croissance | Croissance | Intensité de coloration | Couleur | Croissance | Intensité de coloration | Couleur |
| *Escherichia coli* | ++ | ++ | ++ | orange | ++ | - | - |
| *Serratia marcescens* | ++ | ++ | ++ | orange | ++ | + | orange |
| *Pseudomonas aeruginosa* | ++ | ++ | ++ | orange | ++ | - | - |
| *Burkholderia cepacia* | ++ | ++ | - | - | ++ | - | - |
| *Yersinia enterocolitica* | + | + | ++ | orange | + | - | - |
| *Salmonella ser. typhimurium* | ++ | ++ | - | - | ++ | - | - |
| *Citrobacter freundii* | ++ | ++ | ++ | orange | ++ | - | - |
| *Morganella morganii* | ++ | ++ | ++ | orange | ++ | - | - |
| *Enterobacter cloacae* | ++ | ++ | + | orange | ++ | - | - |
| *Providencia rettgeri* | ++ | ++ | ++ | orange | ++ | - | - |
| *Klebsiella pneumoniae* | ++ | ++ | ++ | orange | ++ | - | - |
| *Acinetobacter baumannii* | ++ | ++ | ++ | orange | ++ | - | - |
| | | | | | | | |
| *Bacillus subtilis* | ++ | ++ | + | orange | ++ | + | orange |
| *Enterococcus faecalis* | + | + | + | orange | + | + | orange |
| *Enterococcus faecium* | + | + | - | - | + | + | orange |
| *Staphylococcus epidermidis* | ++ | ++ | + | orange | ++ | + | orange |
| *Staphylococcus aureus* | ++ | ++ | + | orange | ++ | - | - |
| *MRSA* | ++ | ++ | ++ | orange | ++ | + | orange |
| *Streptococcus pyogenes* | + | + | + | orange | + | - | - |
| *Listeria monocytogenes* | + | + | ++ | orange | + | + | orange |
| | | | | | | | |
| *Candida albicans* | ++ | ++ | - | - | ++ | - | - |
| *Candida glabrata* | + | + | - | - | + | - | - |

| | | | | | | | |
|---|---|---|---|---|---|---|---|
| *Nota bene :* les références internes des souches sont le mêmes que dans l'exemple 3 Légende : ++ : moyenne croissance/intensité de coloration moyenne + : faible croissance/intensité de coloration faible - : pas de croissance/pas de coloration | | | | | | | |

### e) Conclusion

Les composés L-alanyl-4-(4'-amidostyryl)-N-methylquinolinium (dichlorure) et L-alanyl-2-(4'-amidostyryl)-N-methylquinolinium (dichlorure) permettent de détecter une activité alanine-aminopeptidase.

### Exemple 6 - Utilisation de substrats de formule I selon l'invention pour détecter une variation de pH

### a) Indicateurs de pH

Le composé 4-(4'-aminostyryl)-quinoline a été synthétisé comme décrit à l'exemple 1

### b) Préparation du milieu

Une solution-mère à 50g/L en 4-(4'-aminostyryl)-quinoline a été préparée dans un solvant type DMSO et ajoutée dans un milieu chromID coli® modifié préalablement autoclavé et maintenu en surfusion, à raison de 50mg/L. Après homogénéisation, ce milieu a été réparti en boîtes de Petri carrées de 120 mm. Un milieu servant de témoin de croissance (chromID coli® modifié) et noté T a également été coulé.
*Composition du milieu chromID coli® modifié (en g/L) :
Peptones : 5.5
Extrait de levure : 8
Chlorure de sodium : 5
Sels biliaires : 0.7
Glucose : 10
Agar : 12.5

### c) Ensemencement et incubation

Quarante quatre souches de micro-organismes (détails dans le tableau ci-dessous) ont été ensemencées en spots-tests : dépôt de 1 µL à partir de suspensions bactériennes à 0.5McFarland (15.10⁴ CFU/spot)

Les milieux ont été incubés 24 heures à 37°C, puis les spots formés ont été examinés visuellement.

| | **Espèce** | **n°API** |
|---|---|---|
| 1 | *Klebsiella pneumoniae ssp pneumoniae* | 73 08 012 |
| 2 | *Edwarsiella tarda* | 75 07 053 |
| 3 | *Citrobacter koseri* | 75 08 021 |
| 4 | *Shigella spp* | 76 02 055 |
| 5 | *E. coli* | 77 05 035 |
| 6 | *E. coli* | 78 12 004 |
| 7 | *Pectobacterium atrosepticum* | 80 12 075 |
| 8 | *Salmonella ser. Typhimurium* | 81 12 010 |
| 9 | *Pectobacterium carotovorum* | 84 06 059 |
| 10 | *Kluyvera ascorbata* | 84 08 084 |
| 11 | *Escherichia vulneris* | 85 06 777 |
| 12 | *Providencia rettgeri* | 88 05 064 |
| 13 | *Shigella flexneri* | 92 11 059 |
| 14 | *Shigella sonnei* | 92 11 060 |
| 15 | *Yersinia enterocolitica ssp enterocolitica* | 92 11 061 |
| 16 | *Providencia stuartii* | 92 12 101 |
| 17 | *Salmonella spp* | 93 03 001 |
| 18 | *Salmonella ser Enteritidis* | 94 06 001 |
| 19 | *Leclercia adecarboxylata* | 97 04 041 |
| 20 | *Morganella morganii ssp morganii* | 97 08 046 |
| 21 | *Shigella boydii* | 00 04 212 |
| 22 | *Escherichia hermanni* | 04 03 161 |
| 23 | *Proteus mirabilis* | 04 05 048 |
| 24 | *Pantoea agglomerans* | 04 05 057 |
| 25 | *Proteus vulgaris* | 04 05 062 |
| 26 | *Enterobacter sakazakii* | 05 01 020 |
| 27 | *Rahnella aquatilis* | 05 04 103 |
| 28 | *Serratia liquefaciens* | 05 04 117 |
| 29 | *Cedecea lapagei* | 05 04 130 |
| 30 | *Raoultella ornithinolytica* | 05 04 140 |
| 31 | *Yersinia enterocolitica* | 05 04 143 |
| 32 | *E.coli* | 05 04 163 |
| 33 | *Citrobacter freundii* | 05 04 165 |
| 34 | *Buttiauxella agrestis* | 05 04 166 |
| 35 | *Klebsiella oxytoca* | 05 04 175 |
| 36 | *Hafnia alvei* | 05 04 201 |
| 37 | *Enterobacter intermedius* | 05 04 110 |
| 38 | *E. coli* | 05 06 043 |
| 39 | *Ewingella americana* | 05 06 045 |
| 40 | *Enterobacter amnigenus* | 05 06 046 |
| 41 | *E. coli* | 05 06 052 |
| 42 | *Citrobacter braakii* | 05 06 109 |
| 43 | *Enterobacter cloacae* | 05 09 064 |
| 44 | *Pseudomonas aeruginosa* | 89 09 041 |

### d) Lecture des résultats

Les résultats obtenus sont présentés dans le tableau ci-après :

| | T | 4-(4'-aminostyryl)-quinoline | | |
|---|---|---|---|---|
| N°souches | Croissance | Croissance | Intensité de coloration | Couleur |
| 1 | ++ | ++ | ++ | Orange |
| 2 | ++ | ++ | ++ | Orange |
| 3 | ++ | ++ | ++ | Orange |
| 4 | ++ | ++ | ++ | Orange |
| 5 | ++ | ++ | ++ | Orange |
| 6 | ++ | ++ | ++ | Orange |
| 7 | - | - | - | - |
| 8 | ++ | ++ | ++ | Orange |
| 9 | ++ | + | ++ | Orange |
| 10 | ++ | ++ | ++ | Orange |
| 11 | ++ | ++ | ++ | Orange |
| 12 | ++ | ++ | ++ | Orange |
| 13 | ++ | ++ | ++ | Orange |
| 14 | ++ | ++ | ++ | Orange |
| 15 | ++ | ++ | ++ | Orange |
| 16 | ++ | ++ | ++ | Orange |
| 17 | ++ | ++ | ++ | Orange |
| 18 | ++ | ++ | ++ | Orange |
| 19 | ++ | ++ | ++ | Orange |
| 20 | ++ | ++ | ++ | Orange |
| 21 | ++ | ++ | ++ | Orange |
| 22 | ++ | ++ | ++ | Orange |
| 23 | ++ | ++ | ++ | Orange |
| 24 | ++ | ++ | ++ | Orange |
| 25 | ++ | ++ | ++ | Orange |
| 26 | ++ | ++ | - | - |
| 27 | ++ | ++ | ++ | Orange |
| 28 | ++ | ++ | ++ | Orange |
| 29 | ++ | ++ | ++ | Orange |
| 30 | ++ | ++ | ++ | Orange |
| 31 | ++ | + | ++ | Orange |
| 32 | ++ | ++ | ++ | Orange |
| 33 | ++ | ++ | ++ | Orange |
| 34 | ++ | ++ | ++ | Orange |
| 35 | ++ | ++ | ++ | Orange |
| 36 | ++ | ++ | ++ | Orange |
| 37 | ++ | + | ++ | Orange |
| 38 | ++ | ++ | ++ | Orange |
| 39 | ++ | ++ | ++ | Orange |
| 40 | ++ | ++ | ++ | Orange |
| 41 | ++ | ++ | ++ | Orange |
| 42 | ++ | ++ | ++ | Orange |
| 43 | ++ | ++ | ++ | Orange |
| 44 | ++ | + | - | - |

### e) Conclusion

Le 4-(4'-aminostyryl)-quinoline ne présente pas de toxicité particulière.

Son utilisation permet de révéler une acidification via l'apparition d'une coloration orange vif ne diffusant que peu après 24h d'incubation.

Dans les conditions testées, l'utilisation du 4-(4'-aminostyryl)-quinoline permet de détecter les souches capables de fermenter le glucose.

### Exemple 7 - Utilisation de substrats de formule 1 selon l'invention pour détecter une variation de pH

### a) Indicateurs de pH

Le composé 4-(4'-aminostyryl)-quinoline a été synthétisé comme décrit à l'exemple 1

### b) Préparation du milieu

Une solution-mère à 50g/L en 4-(4'-aminostyryl)-quinoline et une solution-mère à 40g/L en Blue-b-D-GUR (soit le 5-bromo-3-indoxyl-b-D-glucuronic acid) ont été préparées dans un solvant type DMSO et ajoutées dans un milieu chromID coli® modifié préalablement autoclavé et maintenu en surfusion, à raison de, respectivement 50mg/L et 200mg/L. Après homogénéisation, ce milieu a été réparti en boîtes de Petri carrées de 120 mm. Un milieu servant de témoin de croissance (chromID coli® modifié) et noté T a également été coulé.
*Composition du milieu chromID coli® modifié (en g/L) :
Peptones : 5.5
Extrait de levure : 8
Chlorure de sodium : 5
Sels biliaires : 0.7
Glucose : 10
Activateur : 0.2
Agar : 12.5

### c) Ensemencement et incubation

Quarante trois souches de micro-organismes (détails dans le tableau ci-dessous) ont été ensemencées en spots-tests : dépôt de 1µL à partir de suspensions bactériennes à 0.5McFarland (15.10⁴ CFU/spot)

Les milieux ont été incubés 48 heures à 37°C, puis les spots formés ont été examinés visuellement.

| | **Espèce** | **n°API** |
|---|---|---|
| 1 | *Klebsiella pneumoniae ssp pneumoniae* | 73 08 012 |
| 2 | *Edwarsiella tarda* | 75 07 053 |
| 3 | *Citrobacter koseri* | 75 08 021 |
| 4 | *Shigella spp* | 76 02 055 |
| 5 | *E. coli* | 77 05 035 |
| 6 | *E. coli* | 78 12 004 |
| 7 | *Pectobacterium atrosepticum* | 80 12 075 |
| 8 | *Salmonella ser. Typhimurium* | 81 12 010 |
| 9 | *Pectobacterium carotovorum* | 84 06 059 |
| 10 | *Kluyvera ascorbata* | 84 08 084 |
| 11 | *Escherichia vulneris* | 85 06 777 |
| 12 | *Providencia rettgeri* | 88 05 064 |
| 13 | *Shigella flexneri* | 92 11 059 |
| 14 | *Shigella sonnei* | 92 11 060 |
| 15 | *Yersinia enterocolitica ssp enterocolitica* | 92 11 061 |
| 16 | *Providencia stuartii* | 92 12 101 |
| 17 | *Salmonella spp* | 93 03 001 |
| 18 | *Salmonella ser. Enteritidis* | 94 06 001 |
| 19 | *Leclercia adecarboxylata* | 97 04 041 |
| 20 | *Morganella morganii ssp morganii* | 97 08 046 |
| 21 | *Shigella boydii* | 00 04 212 |
| 22 | *Escherichia hermannii* | 0403 161 |
| 23 | *Pantoea agglomerans* | 04 05 057 |
| 24 | *Proteus vulgaris* | 04 05 062 |
| 25 | *Enterobacter sakazakii* | 05 01 020 |
| 26 | *Rahnella aquatilis* | 05 04 103 |
| 27 | *Serratia liquefaciens* | 05 04 117 |
| 28 | *Cedecea lapagei* | 05 04 130 |
| 29 | *Raoultella ornithinolytica* | 05 04 140 |
| 30 | *Yersinia enterocolitica* | 05 04 143 |
| 31 | *E. coli* | 05 04 163 |
| 32 | *Citrobacter freundii* | 05 04 165 |
| 33 | *Buttiauxella agrestis* | 05 04 166 |
| 34 | *Klebsiella oxytoca* | 05 04 175 |
| 35 | *Hafnia alvei* | 05 04 201 |
| 36 | *Enterobacter intermedius* | 05 04 110 |
| 37 | *E. coli* | 05 06 043 |
| 38 | *Ewingella americana* | 05 06 045 |
| 39 | *Enterobacter amnigenus* | 05 06 046 |
| 40 | *E. coli* | 05 06 052 |
| 41 | *Citrobacter braakii* | 05 06 109 |
| 42 | *Enterobacter cloacae* | 05 09 064 |
| 43 | *A cinetobacter lwoffi* | 05 04 082 |

### d) Lecture des résultats

Les résultats obtenus sont présentés dans le tableau ci-après.

| | T | 4-(4'-aminostyryl)-quinoline + Blue-b-GUR | | |
|---|---|---|---|---|
| N°souches | Croissance | Croissance | Intensité de coloration | Couleur |
| 1 | ++ | ++ | ++ | orange |
| 2 | ++ | ++ | ++ | orange |
| 3 | ++ | ++ | ++ | orange |
| 4 | ++ | ++ | ++ | orange marron |
| 5 | ++ | ++ | ++ | marron |
| 6 | ++ | ++ | ++ | marron |
| 7 | - | - | - | - |
| 8 | ++ | ++ | ++ | orange |
| 9 | ++ | + | ++ | orange |
| 10 | ++ | ++ | ++ | orange |
| 11 | ++ | ++ | ++ | orange |
| 12 | ++ | ++ | ++ | orange |
| 13 | ++ | ++ | ++ | orange |
| 14 | ++ | ++ | ++ | orange marron |
| 15 | ++ | + | ++ | orange |
| 16 | ++ | ++ | ++ | orange |
| 17 | ++ | ++ | ++ | orange |
| 18 | ++ | ++ | ++ | orange |
| 19 | ++ | ++ | ++ | orange |
| 20 | ++ | ++ | ++ | orange |
| 21 | ++ | ++ | ++ | marron |
| 22 | ++ | ++ | ++ | orange |
| 23 | ++ | ++ | ++ | orange |
| 24 | ++ | ++ | ++ | orange |
| 25 | ++ | ++ | - | - |
| 26 | ++ | ++ | ++ | orange |
| 27 | ++ | ++ | + | orange |
| 28 | ++ | ++ | ++ | orange |
| 29 | ++ | ++ | ++ | orange |
| 30 | ++ | ++ | ++ | orange |
| 31 | ++ | ++ | ++ | marron |
| 32 | ++ | ++ | ++ | orange |
| 33 | ++ | ++ | ++ | orange |
| 34 | ++ | ++ | ++ | orange |
| 35 | ++ | ++ | ++ | orange |
| 36 | ++ | ++ | ++ | orange |
| 37 | ++ | + | ++ | marron |
| 38 | ++ | ++ | ++ | orange |
| 39 | ++ | ++ | ++ | orange |
| 40 | ++ | ++ | ++ | marron |
| 41 | ++ | ++ | ++ | orange |
| 42 | ++ | ++ | ++ | orange |
| 43 | ++ | ++ | ++ | orange |
| 44 | ++ | + | - | - |

### e) Conclusion

Le 4-(4'-aminostyryl)-quinoline associé au Blue-b-GUR permettent de détecter simultanément les entérobactéries (acidification du glucose révélée par la coloration orange du 4-(4'-aminostyryl)-quinolinium) et les souches de *E*. *coli* beta-glucuronidase positive (ainsi que les *Shigella -* d'un point de vue génomique, les souches de *Shigella* appartiennent à l'espèce *E*. *coli*.) par la coloration marron des colonies, mélange de orange et de bleu (suite à l'hydrolyse du Blue-b-GUR).

## Revendications

1. Utilisation d'un composé de la formule (I) suivante, comme substrat enzymatique pour la détection d'une activité peptidase et/ou une variation de pH : 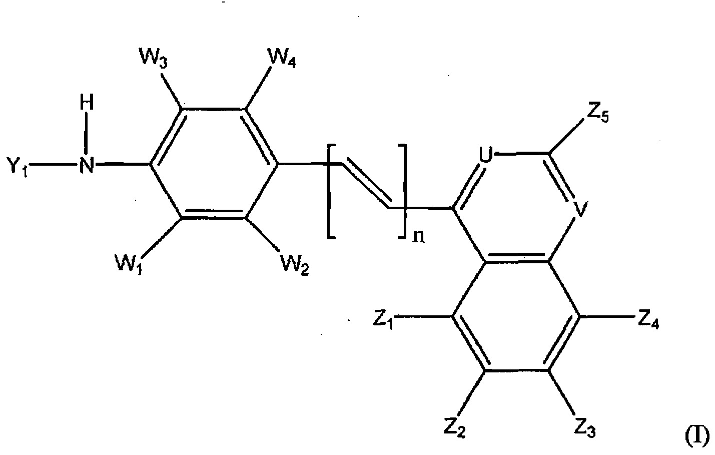 selon laquelle :
• Y₁ est un peptide, H ou un alkyl
• W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci
• n = 0, 1 ou 2
• U est N ou N⁺R et V est CZ₆ N ou N⁺R ou bien V est N ou N⁺R et U est CZ₆,
• R est H, alkyle, aralkyle, aryle, alcanoïque ou alkylsulfonique
• Z₁, Z₂, Z₃, Z₄ et Z₅, sont indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels

2. Utilisation d'un composé de formule I selon la revendication 1 selon laquelle Y₁ est un peptide, préférentiellement choisi parmi l'alanine, l'asparagine, la glutamine, la tyrosine

3. Utilisation d'un composé de formule I selon la revendication 1 ou 2 selon laquelle n = 1

4. Utilisation d'un composé de formule I selon l'une quelconque des revendications 1 à 3 selon laquelle W₁, W₂, W₃ et W₄ sont indépendamment H

5. Utilisation d'un composé de formule I selon l'une quelconque des revendications 1 à 4 selon laquelle U est CH et V est N ou N⁺R, préférentiellement N⁺CH3

6. Utilisation d'un composé de formule I selon l'une quelconque des revendications 1 à 4 selon laquelle V est CH et U est N ou N⁺R, préférentiellement N⁺CH3

7. Utilisation d'un composé de formule 1 selon l'une quelconque des revendications 1 à 6 selon laquelle Z₁, Z₂, Z₃ et Z₄ sont indépendamment H

8. Utilisation d'un composé de formule I selon la revendication 1 selon laquelle ledit composé est choisi parmi : L-Alanyl-4-(4'-amidophenyl)-2-méthyl-quinoline, L-Alanyl-4-(4'-amidostyryl)-N-benzyl-quinolinium TFA, L-Glutamyl-4-(4'-amidostyryl)-N-benzyl-quinolinium TFA, beta-Alanyl-4-(4'-amidostyryl)-N-benzyl-quinolinium TFA, L-Alanyl-4-(4'-amidostyryl)-N-methyl-quinolinium TFA, D-Alanine-N-méthyl-4-(4'-aminostyryl)-quinolinium (dichlorure), L-Alanyl-2-(4'-amidostyryl)-N-méthyl-quinolinium, Z-Alanyl-2-(4'-amidostyryl)-N-méthyl-quinolinium, L-Alanyl-4-(4'-amidostyryl)-N-méthyl-quinolinium (dichlorure), L-Alanyl-L-alanyl-4-(4'-amidostyryl)-N-méthyl-quinolinium (dichlorure), L-Alanyl-L-alanyl-L-alanyl-4-(4'-amidostyryl)-N-méthyl-quinolinium (dichlorure), γ-Aminobutyryl-4-(4'-amidostyryl)-N-methylquinolinium di-Cl, L-Asparaginyl-4-(4'-amidostyryl)-N-méthyl-quinolinium-bis-TFA, α-Aspartyl-4-(4'-amidostyryl)-N-methylquinolinium dichloride, β-Aspartyl-4-(4'-amidostyryl)-N-methylquinolinium dichloride, Glycyl-4-(4'-amidostyryl)-N-méthyl-quinolinium (dichlorure), L-gamma-Glutamyl-4-(4'-amidostyrl)-N-méthyl-quinolinium (dichlorure), Glycyl-4-(4'-amidostyryl)-N-méthyl-quinolinium chloride, L-Leucyl-4-(4'-amidostyryl)-N-méthyl-quinolinium dihydrochloride, L-Methionyl-4-{4'-amidostyryl)-N-methyl-quinolinium di-Cl, Pyroglutamyl-4-(4'-amidostyryl)-N-méthyl-quinolinium iodide, L-Tryptophanyl-4-(4-amidostyryl)-N-méthyl-quinolinium dihydrochloride, L-Tyrosyl-4-(4'-amidostyryl)-N-méthyl-quinolinium dihydrochloride, beta-Alanyl-2-(4'-amidostyryl)-N-methyl-quinolinium TFA, L-Prolyl-2-(4'-amidostyryl)-N-méthyl-quinolinium (HBr), Z-Arginyl-2-(4'-amidostyryl)-N-méthyl-quinolinium, L-Pyroglutamyl -2-(4'-amidostyryl)-N-méthyl-quinolinium, L-Alanyl-4-(4'-amidostyryl)quinoline TFA, ß-Alanyl-4-(4'-amidostyryl)-quinoline, L-Glutamyl-4-(4'-amidostyryl)quinoline TFA, Glycyl-4-(4'-amidostyryl)quinoline TFA, Pyroglutamyl-4-(4'-amidostyryl) quinoline, L-Alanyl-2-(4'-amidostyryl)-quinoline, ß-Alanyl-2-(4'-amidostyryl)-quinoline, Glycyl-2-(4'-amidostyryl)quinoline TFA, L-Alanyl-2-(2'-pyridyl)-4-(4"-amidostyryl)-quinoline

9. Procédé pour la détection chez des micro-organismes d'une activité peptidase et/ou d'une variation de pH, **caractérisé en ce qu'**il comprend les étapes suivantes :
a) Disposer d'un milieu de détection et/ou d'identification comprenant composé de la formule (I) suivante : 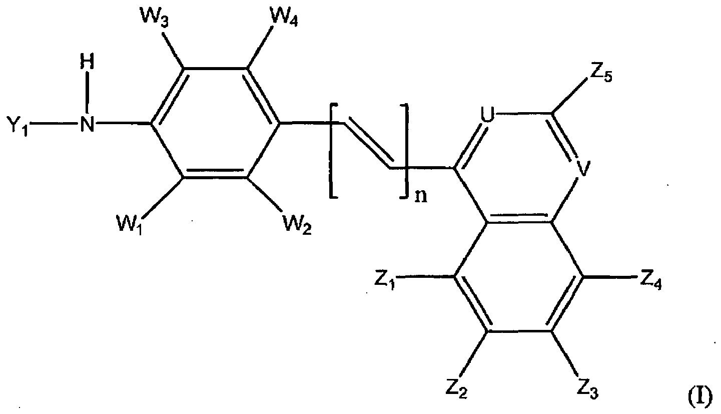 selon laquelle :
• Y₁ est un peptide, H ou un alkyl
• W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci
• n = 0, 1 ou 2
• U est N ou N⁺R et V est CZ₆ N ou N⁺R ou bien V est N ou N⁺R et U est CZ₆,
• R est H, alkyle, aralkyle, aryle, alcanoïque ou alkylsulfonique
• Z₁, Z₂, Z₃, Z₄ et Z₅, sont indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels.
b) ensemencer le milieu avec un échantillon biologique à tester,
c) laisser incuber, et
d) révéler la présence d'au moins une activité peptidase ou une variation de pH

10. Milieu de détection et/ou d'identification de microorganismes comprenant un composé de la formule (I) suivante pour détecter une activité peptidase et/ou une variation de pH : 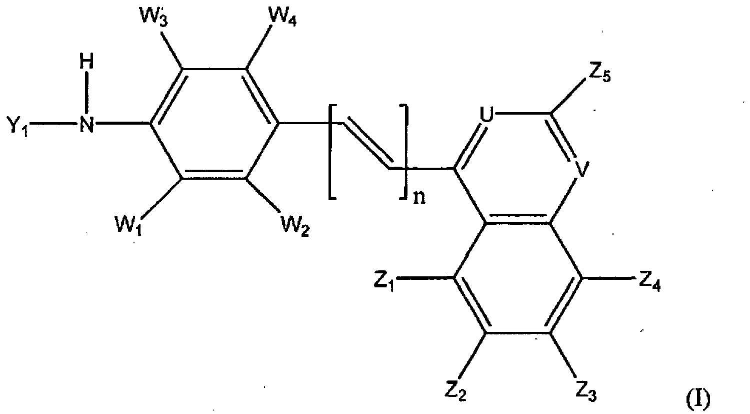 selon laquelle :
• Y₁ est un peptide, H ou un alkyl
• W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci
• n = 0, 1 ou 2
• U est N ou N⁺R et V est CZ₆ N ou N⁺R ou bien V est N ou N⁺R et U est CZ₆,
• R est H, alkyle, aralkyle, aryle, alcanoïque ou alkylsulfonique
• Z₁, Z₂, Z₃, Z₄ et Z₅, sont indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels.

## Patentansprüche

1. Verwendung einer Verbindung der folgenden Formel (I) als Enzymsubstrat zum Nachweis einer Peptidase-Aktivität und/oder einer Veränderung des pH-Werts: 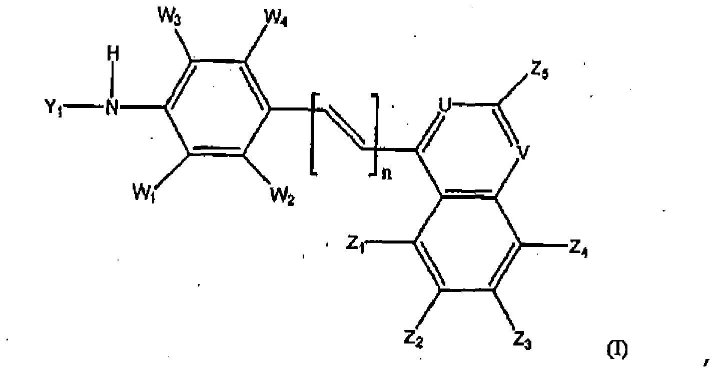 worin:
• Y₁ ein Peptid, H oder ein Alkyl ist
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• U für N oder N⁺R steht und V für CZ₆N oder N⁺R steht oder auch V für N oder N⁺R steht und U für CZ₆ steht,
• R für H, Alkyl, Aralkyl, Aryl, Alkanoyl oder Alkylsulfonyl steht
• Z₁, Z₂, Z₃, Z₄ und Z₅ sind unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl,
und deren Salze.

2. Verwendung einer Verbindung der Formel I nach Anspruch 1, wobei Y₁ ein Peptid ist, das vorzugsweise aus Alanin, Asparagin, Glutamin, Tyrosin ausgewählt wird.

3. Verwendung einer Verbindung der Formel I nach Anspruch 1 oder 2, wobei n = 1.

4. Verwendung einer Verbindung der Formel I nach einem der Ansprüche 1 bis 3, wobei W₁, W₂, W₃ und W₄ unabhängig für H stehen.

5. Verwendung einer Verbindung der Formel I nach einem der Ansprüche 1 bis 4, wobei U für CH steht und V für N oder N⁺R, vorzugsweise N⁺CH₃, steht.

6. Verwendung einer Verbindung der Formel I nach einem der Ansprüche 1 bis 4, wobei V für CH steht und U für N oder N⁺R, vorzugsweise N⁺CH₃, steht.

7. Verwendung einer Verbindung der Formel I nach einem der Ansprüche 1 bis 6, wobei Z₁, Z₂, Z₃ und Z₄ unabhängig für H stehen.

8. Verwendung einer Verbindung der Formel I nach Anspruch 1, wobei diese Verbindung aus Folgenden ausgewählt wird: L-Alanyl-4-(4'-amidophenyl)-2-methylchinolin, L-Alanyl-4-(4'-amidostyryl)N-benzylchinolinium-TFA, L-Glutamyl-4-(4'-amidostyryl)N-benzylchinolinium-TFA, beta-Alanyl-4-(4'-amidostyryl)N-benzylchinolinium-TFA, L-Alanyl-4-(4'-amidostyryl)-N-methylchinolinium-TFA, D-Alanin-N-methyl-4-(4'-aminostyryl)chinolinium (Dichlorid), L-Alanyl-2-(4'-amidostyryl)-N-methylchinolinium, Z-Alanyl-2-(4'-amidostyryl)-N-methylchinolinium, L-Alanyl-4-(4'-amidostyryl)-N-methylchinolinium (Dichlorid), L-Alanyl-L-alanyl-4-(4'-amidostyryl)N-methylchinolinium (Dichlorid), L-Alanyl-L-alanyl-L-alanyl-4-(4'-amidostyryl)-N-methylchinolinium (Dichlorid), γ-Aminobutyryl-4-(4'-amidostyryl)-N-methylchinolinium-di-Cl, L-Asparaginyl-4-(4'-amidostyryl)-N-methylchinolinium-bis-TFA, α-Aspartyl-4-(4'-amidostyryl)-N-methylchinolinium-dichlorid, β-Aspartyl-4-(4'-amidostyryl)-N-methylchinolinium-dichlorid, Glycyl-4-(4'-amidostyryl)-N-methylchinolinium (Dichlorid), L-gamma-Glutamyl-4-(4'amidostyryl)-N-methylchinolinium (Dichlorid), Glycol-4-(4'-amidostyryl)-N-methylchinolinium-chlorid, L-Leucyl-4-(4'-amidostyryl)-N-methylchinolinium-dihydrochlorid, L-Methionyl-4-(4'-amidostyryl)-N-methylchinolinium-di-Cl, Pyroglutamyl-4-(4'-amidostyryl)-N-methylchinolinium-iodid, L-Tryptophanyl-4-(4-amidostyryl)-N-methylchinolinium-dihydrochlorid, L-Tyrosyl-4-(4'-amidostyryl)-N-methylchinolinium-dihydrochlorid, beta-Alanyl-2-(4'-amidostyryl)-N-methylchinolinium-TFA, L-Prolyl-2-(4'-amidostyryl)-N-methylchinolinium (HBr), Z-Arginyl-2-(4'-amidostyryl)-N-methylchinolinium, L-Pyroglutamyl-2-(4'-amidostyryl)-N-methylchinolinium, L-Alanyl-4-(4'-amidostyryl)chinolin-TFA, β-Alanyl-4-(4'-amidostyryl)chinolin, L-Glutamyl-4-(4'-amidostyryl)chinolin-TFA, Glycol-4-(4'-amidostyryl)chinolin-TFA, Pyroglutamyl-4-(4'-amidostyryl)chinolin, L-Alanyl-2-(4'-amidostyryl)chinolin, β-Alanyl-2-(4'-amidostyryl)chinolin, Glycyl-2-(4'-amidostyryl)chinolin-TFA, L-Alanyl-2-(2'-pyridyl)-4-(4"-amidostyryl)chinolin.

9. Verfahren zum Nachweis einer Peptidase-Aktivität und/oder einer Veränderung des pH-Werts in Mikroorganismen, **dadurch gekennzeichnet, dass** es die folgenden Schritte umfasst:
a) Bereitstellen eines Nachweis- und/oder Identifikationsmediums, umfassend eine Verbindung der folgenden Formel (I): 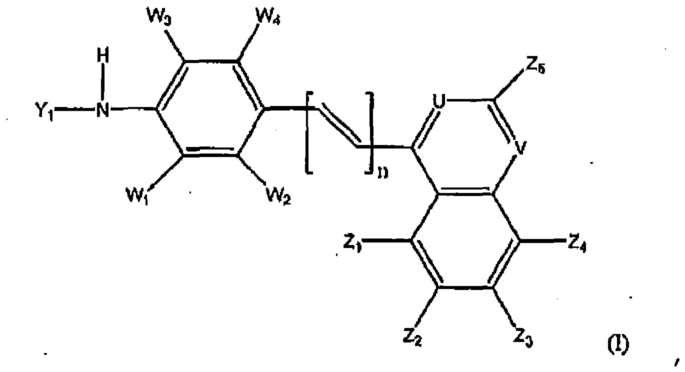 worin:
• Y₁ ein Peptid, H oder ein Alkyl ist
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• U für N oder N⁺R steht und V für CZ₆N oder N⁺R steht oder auch V für N oder N⁺R steht und U für CZ₆ steht,
• R für H, Alkyl, Aralkyl, Aryl, Alkanoyl oder Alkylsulfonyl steht
• Z₁, Z₂, Z₃, Z₄ und Z₅ unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl, ist,
und deren Salze,
b) Beimpfen des Mediums mit einer zu testenden biologischen Probe,
c) Inkubieren und
d) Bestimmen der Anwesenheit von mindestens einer Peptidase-Aktivität oder einer Veränderung des pH-Werts.

10. Medium zum Nachweis und/oder zur Identifikation von Mikroorganismen, umfassend eine Verbindung der folgenden Formel (I) zum Nachweisen einer Peptidase-Aktivität und/oder einer Veränderung des pH-Werts: 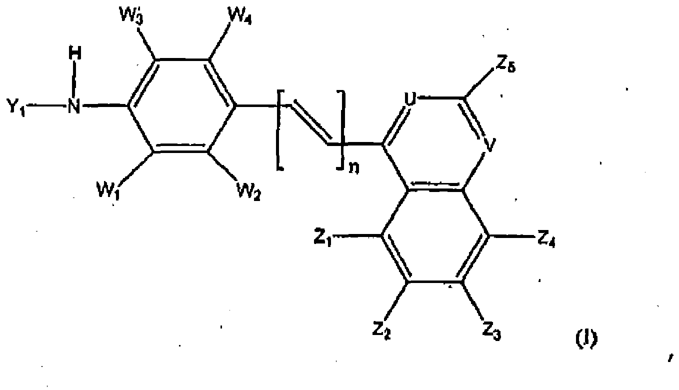 worin:
• Y₁ ein Peptid, H oder ein Alkyl ist
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• U für N oder N⁺R steht und V für CZ₆N oder N⁺R steht oder auch V für N oder N⁺R steht und U für CZ₆ steht,
• R für H, Alkyl, Aralkyl, Aryl, Alkanoyl oder Alkylsulfonyl steht
• Z₁, Z₂, Z₃, Z₄ und Z₅ unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl, ist,
und deren Salze.

## Claims

1. A use of a compound of the following formula I, as an enzyme substrate for the detection of a peptidase activity and/or a variation in pH: 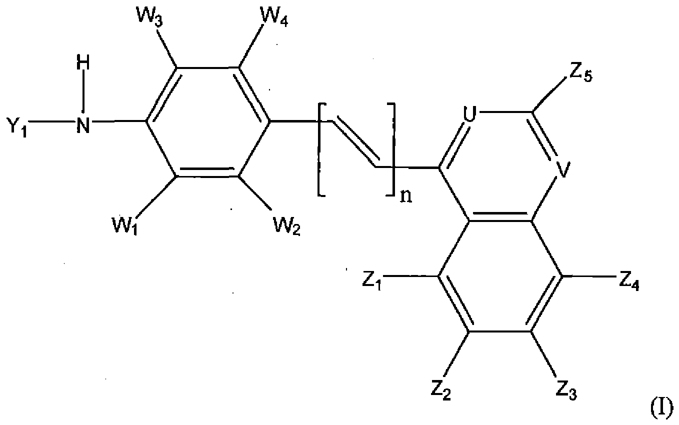 wherein:
• Y₁ is a peptide, H or an alkyl;
• W₁, W₂, W₃ and W₄ are independently H, Br, Cl, F, I, alkyl, alkoxy, thiomethyl, perfluoroalkyl, nitro, cyano, carboxyl (including the esters or amides thereof) or any combination thereof;
• n = 0, 1 or 2;
• U is N or N⁺R and V is CZ₆ N or N⁺R or else V is N or N⁺R and U is CZ₆;
• R is H, alkyl, aralkyl, aryl, alkanoyl or alkylsulfonyl;
• Z₁, Z₂, Z₃, Z₄ and Z₅ are independently H, Br, Cl, F, I, alkyl, aryl, alkoxy, perfluoroalkyl, nitro, cyano, carboxyl, sulfonyl, including the carboxyl or sulfonyl esters or amides,
and salts thereof.

2. The use of a compound of formula I according to claim 1, wherein Y₁ is a peptide, preferentially chosen from alanine, asparagine, glutamine and tyrosine.

3. The use of a compound of formula I according to claim 1 or 2, wherein n = 1.

4. The use of a compound of formula I according to any one of claims 1 to 3, wherein W₁, W₂, W₃ and W₄ are independently H.

5. The use of a compound of formula I according to any one of claims 1 to 4, wherein U is CH and V is N or N⁺R, preferentially N⁺CH₃.

6. The use of a compound of formula I according to any one of claims 1 to 4, wherein V is CH and U is N or N⁺R, preferentially N⁺CH₃.

7. The use of a compound of formula I according to any one of claims 1 to 6, wherein Z₁, Z₂, Z₃ and Z₄ are independently H.

8. The use of a compound of formula I according to claim 1, wherein said compound is chosen from: L-alanyl-4-(4'-amidophenyl)-2-methylquinoline, L-alanyl-4-(4'-amidostyryl)-N-benzylquinolinium TFA, L-glutamyl-4-(4'-amidostyryl)-N-benzylquinolinium TFA, beta-alanyl-4-(4'-amidostyryl)-N-benzylquinolinium TFA, L-alanyl-4-(4'-amidostyryl)-N-methylquinolinium TFA, D-alanine-N-methyl-4-(4'-aminostyryl)quinolinium (dichloride), L-alanyl-2-(4'-amidostyryl)-N-methylquinolinium, Z-alanyl-2-(4'-amidostyryl)-N-methylquinolinium, L-alanyl-4-(4'-amidostyryl)-N-methylquinolinium (dichloride), L-alanyl-L-alanyl-4-(4'-amidostyryl)-N-methylquinolinium (dichloride), L-aianyl-L-alanyl-L-alanyl-4-(4'-amidostyryl)-N-methylquinolinium (dichloride), γ-aminobutyryl-4-(4'-amidostyryl)-N-methylquinolinium di-Cl, L-asparaginyl-4-(4'-amidostyryl)-N-methylquinolinium-bis-TFA, α-aspartyl-4-(4'-amidastyryl)-N-methylquinolinium dichloride, β-aspartyl-4-(4'-amidostyryl)-N-methylquinolinium dichloride, glycyl-4-(4'-amidostyryl)-N-methylquinolinium (dichloride), L-gamma-glutamyl-4-(4'-amidostyryl)-N-methylquinolinium (dichloride), glycyl-4-(4'-amidostyryl)-N-methylquinolinium chloride, L-leucyl-4-(4'-amidostyryl)-N-methylquinolinium dihydrochloride, L-methionyl-4-(4'-amidostyryl)-N-methylquinolinium di-Cl, pyroglutamyl-4-(4'-amidostyryl)-N-methylquinolinium iodide, L-tryptophanyl-4-(4-amidostyryl)-N-methylquinolinium dihydrochloride, L-tyrosyl-4-(4'-amidostyryl)-N-methylquinolinium dihydrochloride, beta-alanyl-2-(4'-amidostyryl)-N-methylquinolinium TFA, L-prolyl-2-(4'-amidostyryl)-N-methylquinolinium (HBr), Z-arginyl-2-(4'-amidostyryl)-N-methylquinolinium, L-pyroglytamyl-2-(4'-amidostyryl)-N-methylquinolinium, L-alanyl-4-(4'-amidostyryl)quinoline TFA, β-alanyl-4-(4'-amidostyryl)quinoline, L-glutamyl-4-(4'-amidostyryl)quinoline TFA, glycyl-4-(4'-amidostyryl)quinoline TFA, pyroglutamyl-4-(4'-amidostyryl)quinoline, L-alanyl-2-(4'-amidostyryl)quinoline, β-alanyl-2-(4'-amidostyryl)quinoline, glycyl-2-(4'-amidostyryl)quinoline TFA and L-alanyl-2-(2'-pyridyl)-4-(4"-amidostyryl)quinoline.

9. A method for the detection in microorganisms of a peptidase activity and/or of a variation in pH, comprising the following steps:
a) providing a detecting and/or identifying medium comprising a compound of the
following formula I: 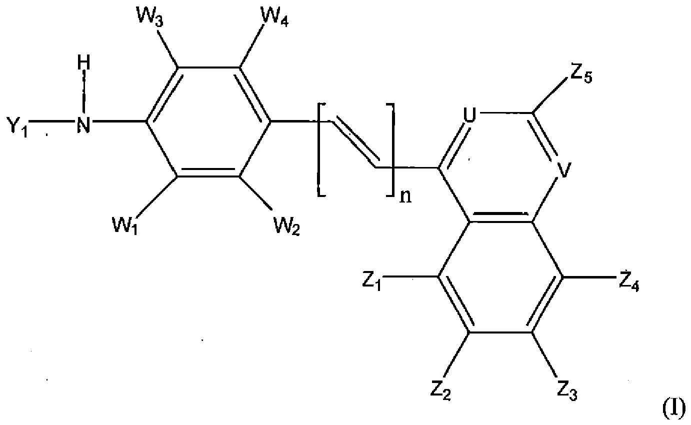 wherein:
• Y₁ is a peptide, H or an alkyl;
• W₁, W₂, W₃ and W₄ are independently H, Br, Cl, F, I, alkyl, alkoxy, thiomethyl, perfluoroalkyl, nitro, cyano, carboxyl (including the esters or amides thereof) or any combination thereof;
• n = 0, 1 or 2;
• U is N or N⁺R and V is CZ₆ N or N⁺R or else V is N or N⁺R and U is CZ₆;
• R is H, alkyl, aralkyl, aryl, alkanoyl or alkylsuffonyl;
• Z₁, Z₂, Z₃, Z₄ and Z₅ are independently H, Br, Cl, F, I, alkyl, aryl, alkoxy, perfluoroalkyl, nitro, cyano, carboxyl, sulfonyl, including the carboxyl or sulfonyl esters or amides,
and salts thereof,
b) inoculating the medium with a biological sample to be tested,
c) leaving to incubate, and
d) revealing the presence of at least one peptidase activity or a variation in pH.

10. A medium for detecting and/or identifying microorganisms comprising a compound of the following formula I for detecting a peptidase activity and/or a variation in pH: 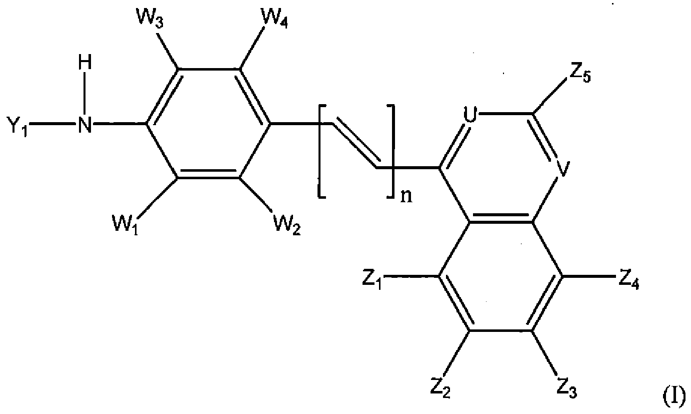 wherein:
• Y₁ is a peptide, H or an alkyl;
• W₁, W₂, W₃ and W₄ are independently H, Br, Cl, F, I, alkyl, alkoxy, thiomethyl, perfluoroalkyl, nitro, cyano, carboxyl (including the esters or amides thereof) or any combination thereof;
• n = 0, 1 or 2;
• U is N or N⁺R and V is CZ₆ N or N⁺R or else V is N or N⁺R and U is CZ₆;
• R is H, alkyl, aralkyl, aryl, alkanoyl or alkylsulfonyl;
• Z₁, Z₂, Z₃, Z₄ and Z₅ are independently H, Br, Cl, F, I, alkyl, aryl, alkoxy, perfluoroalkyl, nitro, cyano, carboxyl, sulfonyl, including the carboxyl or sulfonyl esters or amides,
and salts thereof.
